# EUROPÄISCHE PATENTSCHRIFT

(11) **EP 4 084 758 B1**
(45) Veröffentlichungstag und Bekanntmachung des Hinweises auf die Patenterteilung: **11.09.2024**
(21) Anmeldenummer: 20841945.7
(22) Anmeldetag: 28.12.2020
(51) Int. Cl.: A61F 13/491, A61F 13/494, A61F 13/496

(54) **WEGWERFBARER INKONTINENZARTIKEL IN HÖSCHENFORM**
DISPOSABLE INCONTINENCE ARTICLE IN THE FORM OF BRIEFS
ARTICLE D'INCONTINENCE JETABLE SOUS FORME DE CULOTTE OU SLIP

(30) Priorität: 30.12.2019 DE 102019135887
(43) Veröffentlichungstag der Anmeldung: 09.11.2022
(73) Patentinhaber: Paul Hartmann AG, 89522 Heidenheim (DE)
(72) Erfinder: EILERS, Jörg, 89179 Beimerstetten (DE)
(74) Vertreter: DREISS Patentanwälte PartG mbB
(86) Internationale Anmeldenummer: PCT/EP2020/087909
(87) Internationale Veröffentlichungsnummer: WO 2021/136754

(56) Entgegenhaltungen:
- WO-A1-2019/219943

## Beschreibung

Die Erfindung betrifft einen wegwerfbaren Inkontinenzartikel in Höschenform, also einen Pull-Up-Artikel, für die Aufnahme von Körperausscheidungen für die bevorzugte Verwendung durch Männer. Der Inkontinenzartikel weist eine Längsmittelachse sowie eine Quermittelachse auf und in Längsrichtung voneinander beabstandet einen vorderen Bauchabschnitt und einen hinteren Rückenabschnitt, die zur Bildung eines in Quer- oder Hüftumfangsrichtung durchgehenden Bauch- und Rückenbandes mit einer in Hüftumfangsrichtung geschlossenen Hüftöffnung an beidseitigen Seitennahtbereichen herstellerseitig miteinander verbunden sind und mit einem einen Absorptionskörper aufweisenden Schrittabschnitt, der sich in einer Längsrichtung zwischen Bauchabschnitt und Rückenabschnitt erstreckt und an den Bauchabschnitt und den Rückenabschnitt in einem jeweiligen Überlappungsbereich unlösbar angefügt ist, wobei der Schrittabschnitt, der Bauchabschnitt und der Rückenabschnitt gemeinsam Beinöffnungen des Inkontinenzartikels begrenzen und wobei der Schrittabschnitt beidseits eine seitliche Auslaufsperre bildende und beidseits entlang einer Längserstreckung des Absorptionskörpers verlaufende Cuffelemente aufweist, die zumindest entlang einer Cuffsockellinie an einer körperzugewandten Seite des Inkontinenzartikels festgelegt sind und einen unbefestigten freien Längsrand aufweisen, der zumindest abschnittsweise in der Längsrichtung elastifiziert ist, mit dem sich die Cuffelemente von der körperzugewandten Seite des Inkontinenzartikels emporerheben können und so die jeweilige seitliche Auslaufsperre bilden und wobei der Absorptionskörper so angeordnet ist, dass eine Quermittellinie des Absorptionskörpers zwischen der Quermittelachse des Inkontinenzartikels und einer die Hüftöffnung begrenzenden Hüftöffnungskante des Bauchabschnitts angeordnet ist und wobei die Cuffelemente mit variierendem Abstand der Cuffsockellinien voneinander geführt sind und wobei sich der Absorptionskörper in Richtung des Rückenabschnitts über die Quermittelachse des Inkontinenzartikels in Richtung einer Hüftöffnungskante des Rückenabschnitts erstreckt.

Aus dem Stand der Technik sind eine Vielzahl von genderspezifischen Inkontinenzartikeln bekannt.

So offenbart beispielsweise die WO 2005/044169 A1 ein wegwerfbares absorbierendes Inkontinenzprodukt für Männer, das einen nach vorne verschobenen Absorptionskörper aufweist, um eine Anpassung an die männliche Anatomie und den Ort des Flüssigkeitsanfalls bereitzustellen.

Darüber hinaus ist beispielsweise aus der WO 2004/004617A1 ein Männerprodukt bekannt, bei dem der Saugkörper eine zum Schritt hin schmaler werdende Form aufweist und sich bei Benutzung bis knapp unter die Penisregion erstreckt. Ein derartiges Produkt soll für leichte Inkontinenz bzw. Tropfinkontinenz Verwendung finden. Dabei ist am schmalen Ende des Saugkörpers eine Flüssigkeitsbarriere angebracht, um ein Auslaufen zu verhindern.

Weitere genderspezifische Inkontinenzartikel sind aus der WO 2019/219943 A1 bekannt, die sich mit Inkontinenzartikeln in Höschenform befasst für eine bevorzugte Verwendung durch Männer, bei denen ein unbefestigter freier Längsrand der Cuffelemente zum vorderen Bauchabschnitt einen nicht elastifizierten freien Längsrandendabschnitt aufweist.

Der erfindungsgemäße Inkontinenzartikel in Höschenform soll als genderspezifisches Produkt für den männlichen Träger weiterentwickelt werden.

Der Inkontinenzartikel soll gegenüber dem Bekannten noch besser an die männliche Anatomie angepasst werden, insbesondere für Tropfinkontinenz und/oder die leichte Inkontinenz und/oder die mittlere Inkontinenz und/oder insbesondere für einen mobilen Träger vorgesehen sein. Der Anwendungsfall des Inkontinenzartikels erstreckt sich insbesondere nicht bevorzugt auf bettlägerige, immobile Verwender oder Fälle schwerer Inkontinenz.

Die Erfindung löst diese Aufgabe durch einen Inkontinenzartikel mit den Merkmalen des Anspruchs 1 und bildet diesen durch die nachfolgenden Unteransprüche weiter.

Ebenfalls wird die Erfindung durch eine Anordnung gemäß Anspruch 15 gelöst und durch die nachfolgenden Unteransprüche weitergebildet.

Dabei beträgt ein Verhältnis einer Länge S2 des Absorptionskörpers hinter der Quermittelachse des Inkontinenzartikels in Richtung Rückenabschnitt zu einer Länge S1 des Absorptionskörpers vor der Quermittelachse des Inkontinenzartikels in Richtung Bauchabschnitt, als Quotient S2/S1, höchstens 0,30. Hiermit kann bei gutem und unauffälligen Tragekomfort der Inkontinenz dennoch Rechnung getragen werden.

Der Inkontinenzartikel in Höschenform in der vorliegenden Erfindung ist synonym für einen "Pull-up-Artikel" oder "Pants-Artikel oder eine Windelhose zu verstehen. Der Inkontinenzartikel in Höschenform kann aber insbesondere entlang einer Sollbruchlinie, insbesondere in einem Seitennahtbereich auftrennbar sein, beispielsweise zum Abnehmen eines gebrauchten Inkontinenzartikels.

Die Erfindung betrifft grundsätzlich einen wegwerfbaren Inkontinenzartikel in Höschenform mit den Merkmalen des Oberbegriffs, bei dem die Cuffelemente mit variierendem Abstand der Cuffsockellinien voneinander geführt sind, und wobei sich der Absorptionskörper in Richtung des Rückenabschnitts über die Quermittelachse des Inkontinenzartikels in Richtung der Hüftöffnungskante des Rückenabschnitts erstreckt.

Der erfindungsgemäße Inkontinenzartikel weist gegenüber einer Vielzahl von Inkontinenzartikeln einen kürzeren Saugkörper auf, der im Wesentlichen in der vorderen Produkthälfte des Inkontinenzartikels positioniert ist, so dass eine anatomiegerechte Positionierung des Saugkörpers für einen männlichen Träger erreicht werden kann. Insbesondere in den vorgesehenen Anwendungsfällen von schwacher Inkontinenz und/oder leichter Inkontinenz und/oder mittlerer Inkontinenz reicht ein solcher Saugkörper aus, da nicht mit einem signifikanten Flüssigkeitsanfall im zentralen Schrittbereich bei der männlichen Anatomie zu rechnen ist. Mit einer solchen Positionierung des Saugkörpers kann vorteilhaft ein Inkontinenzartikel als Tagprodukt bereitgestellt werden, da nicht mit einem Flüssigkeitstransfer weit in den rückenseitigen Bereich des Inkontinenzartikels gerechnet werden muss, wie es ansonsten bei einer Anwendung als Nachtprodukt oder bei bettlägerigen Nutzern der Fall sein könnte. Eine derartige Positionierung des Absorptionskörpers sorgt dann insbesondere im Rückenbereich für eine unterwäscheähnlichere Optik und ermöglicht somit insbesondere für den mobilen Anwender ein diskreteres Tragen. Darüber hinaus wird gegenüber herkömmlichen Inkontinenzvorlagen ein einfacher und sicherer Gebrauch realisiert, da eine auf die männliche Anatomie abgestimmte bevorzugte Vorpositionierung des Absorptionskörpers im Inkontinenzartikel herstellerseitig bereitgestellt ist. Ferner ist vorgesehen, dass sich der Absorptionskörper gleichwohl geringfügig in den Bereich hinter der Quermittelachse des Inkontinenzartikels in Richtung Rückenabschnitt erstreckt, um einen Flüssigkeitsaustritt sicher zu verhindern. Mit Cuffelementen, deren Cuffsockellinien in einem variierenden Abstand voneinander geführt sind, lassen sich vorteilhaft der männlichen Anatomie angepasste Auslaufsperren bereitstellen, so dass das männliche Genital von den emporerhebbaren Bereichen des Cuffelements taschenartig oder manschettenartig umschlossen werden kann.

Bei einem Inkontinenzartikel mit einem modularen Aufbau aus den Komponenten Bauchabschnitt, Rückenabschnitt und Schrittabschnitt kann der Schrittabschnitt mit Absorptionskörper und Cuffelementen mit Cuffsockellinien im voneinander variierenden Abstand entsprechend angefertigt werden und durch dessen Platzierung in Bezug zu Bauchabschnitt und Rückenabschnitt in einer einfachen Weise eine als Männerprodukt spezifizierte Windelhose bereitgestellt werden.

Der Inkontinenzartikel in Höschenform ist insbesondere ein Erwachseneninkontinenzprodukt. Der Inkontinenzartikel ist insbesondere ein Männer-Inkontinenzartikel in Höschenform.

Insbesondere ist der Inkontinenzartikel ein Inkontinenzartikel für Tropfinkontinenz und/oder leichte Inkontinenz und/oder mittlere Inkontinenz. Insbesondere ist der Inkontinenzartikel für einen mobilen Träger vorgesehen und/oder insbesondere als Inkontinenzartikel für dessen Anwendung als Tag-Produkt. Der Anwendungsfall des Inkontinenzartikels erstreckt sich insbesondere nicht auf bettlägerige Verwender und/oder auf Verwendung als Nachtprodukt und/oder auf Fälle von schwerer Inkontinenz.

Unter Cuffsockellinie wird die Verbindungsstelle zwischen einem emporerhebbaren Bereich des Cuffelements und einer körperzugewandten Lage des Schrittabschnitts verstanden.

Die Cuffsockellinie ergibt sich vorzugsweise aus einem oder mehreren Ultraschallschweißbereichen und/oder aus einem oder mehreren adhäsiven Verbindungsbereichen. Alternativ kann die Cuffsockellinie auch als eine vollflächige Linie an Ultraschallverschweißung und/oder aus Kleber vorgesehen sein.

Der unbefestigte freie Längsrand des Cuffelements ist in distaler/proximaler Anordnung der der Cuffsockellinie gegenüberliegende freie Endbereich eines Cuffelements.

Der Abstand der Cuffsockellinien voneinander wird als Distanz in der Querrichtung des Inkontinenzartikels, zwischen den beidseits sich in Längsrichtung erstreckenden Cuffsockellinien und dabei zwischen jeweils zur Längsmittelachse nächstgelegenen Innenkanten der Cuffsockellinie im plan ausgelegten Zustand des Inkontinenzartikels verstanden.

Vorzugsweise weist die jeweilige Cuffsockellinie einen in Querrichtung, insbesondere bogenförmig, nach innen in Richtung auf eine Längsmittelachse des Inkontinenzartikels und damit in Richtung auf den freien Längsrand des jeweiligen Cuffelements zu verlaufenden vorderen und/oder hinteren Cuffsockellinienendabschnitt auf, wobei dieser vordere und/oder hintere Cuffsockellinienendabschnitt den emporerhebbaren Bereich des Cuffelements in der Längsrichtung begrenzt. Hierdurch wird vorteilhaft eine Taschenbildung befördert, indem die Cuffelemente nach vorne und/oder nach hinten hin verschlossen sind. Gerade bei Verwendung eines Inkontinenzartikels für Männer ist eine derartige Gestaltung vorteilhaft, da durch die Tasche das männliche Genital fixiert werden kann und auch austretende Flüssigkeit besonders gut aufgefangen werden kann.

Vorzugsweise weist das Cuffelement einen vorderen und einen hinteren Cuffsockellinienendabschnitt auf.

Unter "Vorderseite", "vorne" oder "vorderer" soll eine Richtung verstanden werden, die in Richtung Bauch eines Trägers weist, also nach anterior oder ventral. Unter "Hinterseite", "Rückseite", "hinten" oder "hinteren" soll eine Richtung verstanden werden, die in Richtung Rücken eines Trägers weist, also nach posterior oder dorsal.

Die beiden Cuffsockellinien und/oder die jeweils beiden vorderen Cuffsockellinienendabschnitte und/oder die jeweils beiden hinteren Cuffsockellinienendabschnitte weisen vorzugsweise zueinander einen an der Längsmittelachse des Inkontinenzartikels weitgehend spiegelsymmetrischen Verlauf auf.

Die jeweilige Cuffsockellinie weist vorzugsweise einen sich in Längsrichtung parallel zur Längsmittelachse erstreckenden vorderen Längsabschnitt auf. Vorzugsweise weist die jeweilige Cuffsockellinie einen sich in Längsrichtung parallel zur Längsmittelachse erstreckenden hinteren Längsabschnitt auf. Vorzugsweise weist jede Cuffsockellinie einen vorderen und einen hinteren sich in Längsrichtung parallel zur Längsmittelachse erstreckenden Längsabschnitt auf, wobei insbesondere ein Abstand in Querrichtung zwischen den vorderen Längsabschnitten der Cuffsockellinien größer ist als zwischen den hinteren Längsabschnitten der Cuffsockellinien.

Weiter vorzugsweise weist der parallel zur Längsmittelachse verlaufende vordere Längsabschnitt der Cuffsockellinie eine Erstreckung auf, die mindestens 10%, weiter vorzugsweise mindestens 15%, weiter vorzugsweise mindestens 18%, weiter vorzugsweise höchstens 35%, weiter vorzugsweise höchstens 32%, weiter vorzugsweise höchstens 30% einer Gesamtlängserstreckung C3 des freien Längsrandes des Cuffelements beträgt.

Weiter vorzugsweise weist der parallel zur Längsmittelachse verlaufende hintere Längsabschnitt der Cuffsockellinie eine Erstreckung auf, die mindestens 10%, weiter vorzugsweise mindestens 15%, weiter vorzugsweise mindestens 18%, weiter vorzugsweise höchstens 35%, weiter vorzugsweise höchstens 32%, weiter vorzugsweise höchstens 30% einer Gesamtlängserstreckung C3 des freien Längsrandes des Cuffelements beträgt.

Mit Einbringen von insbesondere eines hinteren Längsabschnitts in die Cuffsockellinie kann eine über eine entsprechende Erstreckung gleichbleibende Cuffhöhe und damit einhergehend die Funktion einer Auffangtasche auch unterhalb der Penisregion bereitgestellt werden im Vergleich zu einer kontinuierlich schräg in Richtung zur Längsmittelachse verlaufenden Cuffsockellinie.

Insbesondere weist der Abstand der Cuffsockellinien voneinander entlang ihres Verlaufes in Längsrichtung ein einziges Maximum des Abstands auf. Vorzugsweise ist das einzige Maximum des Abstands der Cufflinien voneinander in einem vorderen emporerhebbaren Bereich der Cuffelemente vorgesehen. Vorzugsweise ist das Maximums des Abstands der Cuffsockellinien voneinander in der Längsrichtung innerhalb eines Bereiches angeordnet, der ausgehend vom Bauchabschnitt in Richtung Rückenabschnitt und in Bezug auf eine vor der Quermittelachse des Inkontinenzartikels vorhandene Längserstreckung C2 des freien Längsrandes den vorderen zwei Dritteln (2/3) der Längserstreckung C2 entspricht, weiter vorzugsweise der vorderen Hälfte (1/2) der Längserstreckung C2 entspricht.

Insbesondere ist ein Maximum des Abstandes der Cuffsockellinien voneinander in der Längsrichtung innerhalb eines Bereiches angeordnet, der ausgehend vom Bauchabschnitt in Richtung Rückenabschnitt innerhalb einer vorderen Hälfte (1/2) einer Gesamtlängserstreckung C3 des freien Längsrands der Cuffelemente befindet, vorzugsweise innerhalb einer Erstreckung vom Ende eines vorderen Achtels (1/8) bis einschließlich des vierten Achtels (4/8) der Längserstreckung C3 des freien Längsrandes.

Der Terminus "dass der Abstand der Cuffsockellinien voneinander ein Maximum durchläuft", wird so verstanden, dass der Abstand der Cuffsockellinien in Querrichtung entlang des Verlaufs in Längsrichtung zunimmmt, ein Maximum erreicht, und dann wieder abnimmt, und sich die Cuffsockellinien somit wieder einander nähern.

Ein Maximum des Abstandes der Cuffsockellinien voneinander kann sich entweder auf einen Punkt, im Sinne eines Wendepunktes im Verlauf der beiden Cuffsockellinien beziehen. Alternativ kann ein Maximum des Abstandes sich auch über einen Abschnitt der Cuffsockellinien erstrecken.

Insbesondere erstreckt sich ein Maximum des Abstands der Cuffsockellinien voneinander in Längsrichtung über einen Abschnitt mit einer Erstreckung, die mindestens 10%, weiter vorzugsweise mindestens 15%, weiter vorzugsweise mindestens 18%, weiter vorzugsweise höchstens 35%, weiter vorzugsweise höchstens 32%, weiter vorzugsweise höchstens 30% einer Gesamtlängserstreckung C3 des freien Längsrandes des Cuffelements beträgt. Insbesondere ist ein Maximum des Abstands der Cuffsockellinien zueinander zwischen vorderen Längsabschnitten der beiden Cuffsockellinien vorgesehen.

Vorzugsweise ist der variierende Abstand der Cuffsockellinien zueinander derart, dass ein Verhältnis eines mittleren vorderen Cuffsockellinienabstands D(v) in einem vorderem Cuffsockellinienabstandsegment zu einem mittleren hinteren Cuffsockellinienabstand D(h) in einem hinteren Cuffsockellinienabstandsegment mindestens 1,2, insbesondere mindestens 1,3, insbesondere mindestens 1,4, und weiter insbesondere höchstens 2,3, weiter insbesondere höchstens 2,2, weiter insbesondere höchstens 2,1, weiter insbesondere höchstens 2,0, weiter insbesondere höchstens 1,9, weiter insbesondere höchstens 1,8, weiter insbesondere höchstens 1,7 beträgt.

Dies unterstützt vorteilhaft die Erreichung einer guten Auffangleistung bei gleichzeitigem Tragekomfort.

Das vordere Cuffsockellinienabstandsegment und das hintere Cuffsockellinienabstandsegment sind hierbei über eine Halbierung des über eine Gesamtlängserstreckung C3 des freien Längsrands der Cuffelemente und innerhalb der beiden Cuffsockellinien vorhandenen Bereichs definiert. Zur Bestimmung des mittleren vorderen Cuffsockellinienabstands und des mittleren hinteren Cuffsockellinienabstands wird das jeweilige Cuffsockellinienabstandsegment durch Anlegen von neun in Längsrichtung äquidistanten und in Querrichtung verlaufenden imaginären Sektionstrennlinien aufgeteilt, wobei eine Distanz der imaginären Sektionstrennlinie zwischen den beiden Cuffsockellinien jeweils einem einzelnen Cuffsockellinienabstand entspricht, und dann eine Summe der neun einzelnen Cuffsockellinienabstände im vorderen Cuffsockellinienabstandsegment und eine Summe der neun einzelnen Cuffsockellinienabstände im hinteren Cuffsockellinienabstandsegment jeweils durch n= 9 geteilt wird unter Erhalt des mittleren vorderen Cuffsockellinienabstands D(v) und des mittleren hinteren Cuffsockellinienabstands D(h). Daraus wird dann das Verhältnis als Quotient D(v)/D(h) gebildet.

Weiter vorzugsweise beträgt ein Verhältnis eines maximalen Abstands der Cuffsockellinien innerhalb einer vorderen Produkthälfte vor der Quermittelachse des Inkontinenzartikels zu einem Abstand der Cuffsockellinien vermessen auf der Quermittelachse des Inkontinenzartikels mindestens 1,3, insbesondere mindestens 1,4, und insbesondere höchstens 2,0, weiter insbesondere höchstens 1,8. Dies unterstützt vorteilhaft die Erreichung einer guten Auffangleistung bei gleichzeitigem Tragekomfort.

Der Abstand der Cuffsockellinien voneinander weist bevorzugt einen maximalen Abstand von 130 - 160 mm, weiter vorzugsweise 135 mm - 155 mm, weiter vorzugsweise 140 - 150 mm auf, diesen dabei insbesondere zwischen den vorderen Längsabschnitten der Cuffsockellinien. Weiter vorzugsweise beträgt der Abstand der Cuffsockellinien voneinander in einem vom maximalen Abstand verschiedenen Bereich, vorzugsweise zwischen den hinteren Längsabschnitten der Cuffsockellinien 70 - 100 mm, weiter vorzugsweise 75 - 100 mm, weiter vorzugsweise 80 - 100 mm, weiter vorzugsweise 85 - 100 mm, weiter vorzugsweise 90 - 95 mm. Insbesondere beträgt der Abstand der Cuffsockellinien voneinander vermessen auf der Quermittelachse des Inkontinenzartikels 70 - 100 mm, weiter vorzugsweise 75 - 100 mm, weiter vorzugsweise 80 - 100 mm, weiter vorzugsweise 85 - 100 mm, weiter vorzugsweise 90 - 95 mm.

Die freien Längsränder des Cuffelements weisen jeweils eine Gesamtlängserstreckung C3 auf. Vorzugsweise erstreckt sich der jeweilige freie Längsrand des Cuffelements in Längsrichtung ausgehend innerhalb eines Bereiches vor der Quermittelachse des Inkontinenzartikels mit einer Längserstreckung C2 und vorzugsweise erstreckt sich der freie Längsrand des Cuffelements ab der Quermittelachse des Inkontinenzartikels in Richtung Rückenabschnitt mit einer Längserstreckung C4.

Zur Vermessung der Gesamtlängserstreckung C3 des freien Längsrandes des Cuffelements, der Längserstreckung C2 des freien Längsrandes vor der Quermittelachse, der Längserstreckung C4 des freien Längsrandes hinter der Quermittelachse oder einer Längserstreckung C1 eines vorderen nicht elastifizierten freien Längsrandendabschnitts wird die Distanz über die Längsrichtung des Inkontinenzartikels vermessen.

Insbesondere ist vorgesehen, dass die freien Längsränder der Cuffelemente zumindest abschnittsweise, vorzugsweise vollständig parallel zur Längsrichtung verlaufend angeordnet sind. Dies ist im plan ausgebreiteten Zustand des Inkontinenzartikels und damit der Cuffelemente ersichtlich.

Vorzugsweise beträgt ein Verhältnis einer Längserstreckung C4 des unbefestigten freien Längsrandes hinter der Quermittelachse des Inkontinenzartikels in Richtung Rückenabschnitt zu einer Längserstreckung C2 des unbefestigten freien Längsrandes vor der Quermittelachse des Inkontinenzartikels in Richtung Bauchabschnitt höchstens 0,30, insbesondere höchstens 0,25, insbesondere höchstens 0,20 und vorzugsweise mindestens 0,05, vorzugsweise mindestens 0,07, weiter vorzugsweise mindestens 0,10. Indem sich die Cuffelemente über die Quermittelachse des Inkontinenzartikels in Richtung Rückenabschnitt erstrecken wird ein sicheres Auffangen von Flüssigkeit auch im Schwerpunkt des Produktes sichergestellt. Dabei wird aber gleichzeitig vorteilhaft ein zu großer Anteil an offenen Cuffelementen, also an emporerhebbaren Bereichen der Cuffelemente in Richtung Rückenbereich vermieden, um eine taschenartige und das männliche Genital umgebende Anpassungsfähigkeit der Cuffelemente bereitzustellen. Zudem wird damit auch die vorgesehene Verwendung als leichtes und dezentes und wenig auftragendes Inkontinenzprodukt unterstützt.

Eine zumindest abschnittsweise in Längsrichtung vorgesehene Elastifizierung des freien Längsrandes des Cuffelements ist wünschenswert, um ein sicheres Emporerheben und Aufstellen der Cuffelemente zu gewährleisten, und damit ihre Funktion als seitliche Auslaufsperre. Eine Elastifizierung kann insbesondere über eine flächenhafte Elastifizierung der Cuffelemente erfolgen, indem die Cuffelemente in Längsrichtung elastisch dehnbare Flächenmaterialien umfassen. Insbesondere kann vorgesehen sein, dass die Cuffelemente im Bereich ihres freien emporerhebbaren Längsrandes im Wesentlichen fadenförmige Elastifizierungsmittel und/oder streifenförmige Elastifizierungsmittel aufweisen.

Vorzugsweise ist der freie Längsrand des Cuffelements über mindestens 65%, weiter insbesondere mindestens 68%, weiter insbesondere mindesten 70%, weiter insbesondere mindestens 72%, weiter insbesondere mindestens 80%, weiter insbesondere mindestens 88%, weiter insbesondere mindestens 90%, weiter insbesondere mindestens 92%, weiter insbesondere mindestens 95% einer Längserstreckung C3 des freien Längsrandes mit einer Elastifizierung versehen.

Vorzugsweise werden zur Elastifizierung des freien Längsrandes fadenförmige und/oder streifenförmige Elastifizierungsmittel mit einer Vorspannung von 1,5 - 4,0, vorzugsweise 1,5 - 3,5, weiter vorzugsweise 1,5 - 3,0 eingesetzt und/oder mit einer Stärke von 100 - 1200 dtex, weiter vorzugsweise von 200 - 1000 dtex, weiter vorzugsweise 300 - 800 dtex eingesetzt.

In einer bevorzugten Variante, kann der jeweilige unbefestigte freie Längsrand der beiden Cuffelemente zum vorderen Bauchabschnitt hin einen nicht elastifizierten freien Längsrandendabschnitt aufweisen, dessen Längserstreckung C1 wenigstens 8ö, insbesondere mindestens 12%, insbesondere mindestens 15% und insbesondere höchstens 35%, insbesondere höchstens 32%, weiter insbesondere höchstens 30% einer Längserstreckung C2 des unbefestigten freien Längsrands der Cuffelemente in der sich von der Quermittelachse des Inkontinenzartikels zum Bauchabschnitt erstreckenden Produkthälfte beträgt. In diesem Fall kann die Gefahr des Scheuerns und von Hautirritationen in diesem insbesondere für die männliche Anatomie kritischen Bereich reduziert werden.

Insbesondere ist vorgesehen, dass der jeweilige unbefestigte freie Längsrand der beiden Cuffelemente zum vorderen Bauchabschnitt hin einen nicht elastifizierten freien Längsrandendabschnitt aufweist, dessen Längserstreckung C1 wenigstens 8ö, insbesondere wenigstens 10% und insbesondere wenigstens 12% sowie insbesondere höchstens 35%, weiter insbesondere höchstens 32%, weiter insbesondere höchstens 30%, weiter insbesondere höchstens 28%, weiter insbesondere höchstens 25% einer Gesamtlängserstreckung C3 des unbefestigten freien Längsrands der Cuffelemente beträgt.

Die Cuffhöhe wird als der Abstand eines distalen Endes der emporerhebbaren Bereiche der Cuffelemente, also des freien Längsrandes von der Cuffsockellinie verstanden. Der Abstand wird als die effektive Länge in Querrichtung bei flachgelegtem, plan ausgebreitetem Cuffelement verstanden.

Vorzugsweise beträgt ein Verhältnis einer innerhalb eines vorderen Längsrand-Cuffsegments berechneten mittleren vorderen Cuffhöhe F(v) zu einer innerhalb eines hinteren Längsrand-Cuffsegments berechneten mittleren hinteren Cuffhöhe F(h) mindestens 1,2, insbesondere mindestens 1,3, insbesondere mindestens 1,4 und insbesondere höchstens 2,0, weiter insbesondere höchstens 1,9, weiter insbesondere höchstens 1,8. Das vordere Längsrand-Cuffsegment und das hintere Längsrand-Cuffsegment werden über eine Halbierung des jeweiligen unbefestigten freien Längsrands der beiden Cuffelemente und den zugeordneten Bereichen der Cuffelemente zwischen Cuffsockellinie und freiem Längsrand des Cuffelements definiert. Der unfestigte freie Längsrand der beiden Cuffelemente weist dabei jeweils eine Gesamtlängserstreckung C3 auf. Zur Bestimmung der mittleren vorderen Cuffhöhe innerhalb des vorderen Längsrand-Cuffsegments und der mittleren hinteren Cuffhöhe innerhalb des hinteren Längsrand-Cuffsegments wird das jeweilige Längsrand-Cuffsegment in Längsrichtung durch Anlegen von neun in Längsrichtung äquidistanten und in Querrichtung verlaufenden imaginären Sektionstrennlinien aufgeteilt, wobei die Sektionstrennlinien den unbefestigten freien Längsrand und die Cuffsockellinie schneiden, und ein jeweiliger Abstand entlang und auf der Sektionstrennlinie jeweils als einzelne Cuffhöhe vermessen wird, und eine Summe der neun einzelnen Cuffhöhen im vorderen Längsrand-Cuffsegment und eine Summe der neun einzelnen Cuffhöhen im hinteren Längsrand-Cuffsegment jeweils durch n= 9 geteilt wird unter Erhalt der mittleren vorderen Cuffhöhe F(v) und der mittleren hinteren Cuffhöhe F(h). Daraus wird das Verhältnis als Quotient F(v)/F(h) gebildet.

Mit einer solchen Cuffhöhen-Verteilung wird vorteilhaft eine gute Taschenbildung für die Verwendung des Inkontinenzartikels als Männerprodukt bereitgestellt.

Bevorzugt beträgt eine maximale Cuffhöhe gemessen in Querrichtung als ein maximaler Abstand eines unbefestigten freien Längsrandes des Cuffelements von der Cuffsockellinie 45 - 75 mm, insbesondere 50 - 70 mm, weiter insbesondere 55 - 70 mm, weiter insbesondere 60 - 70 mm.

Weiter vorzugsweise beträgt eine Cuffhöhe, gemessen bei der Quermittelachse des Inkontinenzartikels, als ein Abstand eines unbefestigten freien Längsrandes der Cuffelemente von der Cuffsockellinie 25 - 55 mm, insbesondere 30 - 50 mm, weiter insbesondere 30 - 45 mm.

Mit solchen Cuffhöhen kann für die Verwendung des Inkontinenzartikels als Männerprodukt eine gute Taschenbildung bereitgestellt werden.

Der Absorptionskörper weist eine Gesamtlängserstreckung S3 auf. Erfindungsgemäß erstreckt sich der Absorptionskörper über die Quermittelachse des Inkontinenzartikels in Richtung Rückenabschnitt, deren Erstreckung als S2 bezeichnet wird, und die Erstreckung des Absorptionskörpers vor der Quermittelachse in Richtung Bauchabschnitt als S1 bezeichnet wird.

Weiter ist der Absorptionskörper entlang einer Längserstreckung beidseits durch Längskanten begrenzt, wobei die Längskanten sich in Längsrichtung des Inkontinenzartikels erstrecken. "In Längsrichtung des Inkontinenzartikels" bedeutet hierbei, dass die Längskanten zumindest eine Erstreckung in Längsrichtung des Inkontinenzartikels aufweisen, die mithin auch schräg oder gekrümmt zur Längsrichtung des Inkontinenzartikels verlaufen kann. Die Längskanten bestimmen dabei die in Längsrichtung verlaufende Kontur des Absorptionskörpers.

Insbesondere kann vorgesehen sein, dass die Längskanten des Absorptionskörpers mit variierendem Abstand voneinander verlaufen, und der Abstand der Längskanten voneinander in Längsrichtung ein Maximum in einem vorderen Bereich des Absorptionskörpers aufweist.

Insbesondere ist ein Maximum des Abstandes der Längskanten des Absorptionskörpers voneinander innerhalb eines Bereiches angeordnet, der ausgehend vom Bauchabschnitt in Richtung Rückenabschnitt und in Bezug auf eine vor der Quermittelachse des Inkontinenzartikels vorhandenen Längserstreckung S1 des Absorptionskörpers den vorderen zwei Drittel (2/3) der Längserstreckung S1 entspricht, weiter vorzugsweise der vorderen Hälfte (1/2) der Längserstreckung S1 entspricht.

Insbesondere ist ein Maximum des Abstandes der Längskanten des Absorptionskörpers voneinander in der Längsrichtung innerhalb eines Bereiches angeordnet, der ausgehend vom Bauchabschnitt in Richtung Rückenabschnitt sich innerhalb einer vorderen Hälfte (1/2) einer Gesamtlängserstreckung S3 des Absorptionskörpers befindet, insbesondere innerhalb einer Erstreckung vom Ende eines vorderen Achtels (1/8) bis einschließlich des vierten Achtels (4/8) der Längserstreckung S3.

Besonders bevorzugt ist eine Glühbirnenform des Absorptionskörpers, der hierdurch besonders funktionsgerecht ausgestaltet ist und bei gleichzeitig größtmöglichen dezenten Trageeigenschaften, sowohl optisch als auch vom Tragegefühl und -komfort. Ein solcher Absorptionskörper weist daher gegenüber einem z.B. dreieckigen Absorptionskörper vorteilhafte Absorptionseigenschaften und eine vorteilhafte Geometrie auf.

Weiter vorzugsweise beträgt ein Verhältnis einer innerhalb eines vorderen Absorptionskörpersegments berechneten mittleren vorderen Absorptionskörperbreite G(v) zu einer innerhalb eines hinteren Absorptionskörpersegments berechneten mittleren hinteren Absorptionskörperbreite G(h) insbesondere mindestens 1,2, insbesondere mindestens 1,3, insbesondere mindestens 1,4 und insbesondere höchstens 2,0, weiter insbesondere höchstens 1,9, weiter insbesondere höchstens 1,8, wobei das vordere und hintere Absorptionskörpersegment durch eine Halbierung einer Gesamtlänge S3 des Absorptionskörpers definiert ist. Zur Bestimmung der mittleren vorderen und mittleren hinteren Absorptionskörperbreite wird das jeweilige vordere und hintere Absorptionskörpersegment in Längsrichtung durch Anlegen von neun in Längsrichtung äquidistanten und in Querrichtung verlaufenden imaginären Sektionstrennlinien aufgeteilt. Ein Abstand zwischen den Längsrändern des Absorptionskörpers entlang und auf jeder der neun Sektionstrennlinien wird jeweils als einzelne Absorptionskörperbreite vermessen und eine Summe der einzelnen Absorptionskörperbreiten des vorderen und des hinteren Absorptionskörpersegments jeweils durch n= 9 geteilt und damit die mittlere vordere Absorptionskörperbreite G(v) und die mittlere hintere Absorptionskörperbreite G(h) berechnet. Daraus wird dann das Verhältnis als Quotient G(v)/G(h) gebildet.

Vorzugsweise ist ein maximaler Abstand der Längskanten des Absorptionskörpers als die maximale Absorptionskörperbreite innerhalb der vorderen Produkthälfte angeordnet.

Bevorzugt beträgt ein maximaler Abstand der Längskanten des Absorptionskörpers, also eine maximale Absorptionskörperbreite, vorzugsweise innerhalb eines vorderen Bereichs des Absorptionskörpers, weiter vorzugsweise innerhalb eines Bereiches des Absorptionskörpers, der ausgehend vom Bauchabschnitt in Richtung Rückenabschnitt einer vorderen Hälfte (1/2) einer Gesamtlängserstreckung S3 des Absorptionskörpers entspricht, 150 - 210 mm, weiter insbesondere 160 - 200, weiter insbesondere 170 - 190 mm.

Weiter vorzugsweise beträgt der Abstand der Längskanten des Absorptionskörpers vermessen auf der Quermittelachse des Inkontinenzartikels 80 - 140 mm, insbesondere 90 -130 mm, weiter insbesondere 100 - 120 mm.

Der Absorptionskörper weist vorzugsweise eine Gesamtlängserstreckung S3 von 280 - 400 mm, weiter insbesondere 300 - 370 mm, weiter insbesondere 300 - 360 mm auf.

Vorzugsweise beträgt ein Verhältnis einer Länge S2 des Absorptionskörpers hinter der Quermittelachse des Inkontinenzartikels in Richtung Rückenabschnitt zu einer Länge S1 des Absorptionskörpers vor der Quermittelachse des Inkontinenzartikels in Richtung Bauchabschnitt, als Quotient S2/S1, höchstens 0,25, insbesondere höchstens 0,20 und weiter vorzugsweise mindestens 0,05, vorzugsweise mindestens 0,10. Hiermit kann bei gutem und unauffälligen Tragekomfort der Inkontinenz dennoch Rechnung getragen werden.

Insbesondere beträgt ein Verhältnis einer Länge S1 des Absorptionskörpers vor der Quermittelachse des Inkontinenzartikels in Richtung Bauchabschnitt zu einer Länge L1 des Inkontinenzartikels ausgehend von der Quermittelachse des Inkontinenzartikels bis zu einer die Hüftöffnung begrenzenden Hüftöffnungskante des Bauchabschnitts, also der Produktlängshälfte, ausgedrückt als Quotient S1/L1, mindestens 0,6, insbesondere mindestens 0,7, weiter vorzugsweise höchstens 0,9, weitere vorzugsweise höchstens 0,8.

Die freien Längsränder der Cuffelemente können insbesondere in Längsrichtung einen Abstand E voneinander, gemessen in Querrichtung, aufweisen.

Vorzugsweise beträgt der Abstand E höchstens 35 mm, weiter vorzugsweise höchstens 30 mm, weiter vorzugsweise höchstens 25 mm, weiter vorzugsweise höchstens 20 mm. Es kann auch vorgesehen sein, dass die freien Längsränder der Cuffelemente ohne Abstand zueinander, also Stoß an Stoß verlaufen. Alternativ kann eine Überlappung E` der freien Längsränder der Cuffelemente vorgesehen sein, dabei mit einer Überlappung E` von insbesondere höchstens 15 mm, weiter insbesondere höchstens 10 mm.

Ein geringer Abstand E ergibt vorteilhaft eine abdichtende Funktionalität der Cuffelemente in deren distalen freien Enden.

In einer bevorzugten Ausführung beträgt ein Verhältnis eines Abstands E zwischen den nach der Fixierung in einem fixierten Endrandabschnitt verbliebenen freien Kanten der Cuffelemente , insbesondere am vorderen zum Bauchabschnitt gerichteten Ende der Cuffelemente, zu einem dem fixierten Endrandabschnitt nächstgelegenen maximalen Abstand der Cuffsockellinien insbesondere höchstens 0,5, weiter insbesondere höchstens 0,4, weiter insbesondere höchstens 0,3. Hierdurch wird eine hinreichend große Tasche mit einer abdichtenden Funktionalität gebildet.

In einer alternativen Ausführung beträgt ein Verhältnis einer Überlappung E` zwischen den nach der Fixierung in einem fixierten Endrandabschnitt verbliebenen freien Kanten der Cuffelemente, insbesondere am vorderen zum Bauchabschnitt gerichteten Ende der Cuffelemente, zu einem dem fixierten Endrandabschnitt nächstgelegenen maximalen Abstand der Cuffsockellinien insbesondere höchstens 0,3, weiter insbesondere höchstens 0,2.

Als vorteilhaft wurde erkannt, ein Verhältnis einer maximalen Höhe der Cuffelemente, also maximalen Cuffhöhe, ausgehend von der Cuffsockellinie zum freien Längsrand zu einer auf der Quermittelachse des Inkontinenzartikels gemessener Breite des Absorptionskörpers von mindestens 0,35, insbesondere mindestens 0,40, insbesondere mindestens 0,45 und insbesondere mindestens 0,50, weiter insbesondere höchstens 0,75, insbesondere höchstens 0,70, weiter insbesondere höchstens 0,65.

Bevorzugt ist vorgesehen, wenn die Cuffsockellinien zumindest abschnittsweise der Kontur der Längskanten des Absorptionskörpers folgen. Insbesondere ist vorgesehen, dass die Cuffsockellinien und die Längskanten des Absorptionskörpers parallel verlaufen. Insbesondere verlaufen die Cuffsockellinien zumindest abschnittsweise, bevorzugt vollständig innerhalb der Kontur des Absorptionskörpers.

Vorzugsweise weist der Schrittabschnitt außerhalb des Absorptionskörpers entlang der Beinöffnungen erstreckte Schrittelastifizierungsmittel auf. Die Schrittelastifizierungsmittel dienen vorteilhaft als zusätzliche seitliche Auslaufsperre und gleichzeitig wird das Anlegen des Schrittabschnitts gegen den Körper eines Trägers und eine bootförmige Krümmung des Schrittabschnitts gefördert und hervorgerufen. Hierdurch wird in Zusammenwirken mit den Elastifizierungsmitteln der Cuffelemente das Aufstellen der Cuffelemente und ihr Emporerheben in Richtung auf die Körperoberfläche befördert.

Vorzugsweise erstrecken sich die Schrittelastifizierungsmittel mit einem vorderen und einem hinteren aktiven Ende mit ihrem hinteren aktiven Ende weiter in Richtung des Rückenabschnitts als ein hinteres Ende des elastifizierten Abschnitts des freien Längsrandes der Cuffelemente. Solchenfalls können die Schrittelastifizierungsmittel auch in diesem Bereich ein Aufstellen des Schrittabschnitts im rückseitigen Bereich unterstützen und einen Abschluss um die Beinöffnung bereitstellen.

Insbesondere bevorzugt ist vorgesehen, dass benachbart zum Längsende des Absorptionskörpers in Quer- oder Hüftumfangsrichtung elastisch dehnbare Bereiche im Bauchabschnitt ausgebildet sind, insbesondere dass der Schrittabschnitt einen in Richtung Bauchabschnitt und/oder Rückenabschnitt über ein Längsende des Absorptionskörpers in Längsrichtung hinauserstreckenden Überhang an Topsheet- und/oder Backsheet-Materiallagen aufweist, über welchen in Quer- oder Hüftumfangsrichtung elastisch dehnbare Bereiche im Bauchabschnitt ausgebildet sind. Hierdurch wird die Taschenbildung der Cuffelemente weiter gefördert, da eine bootartige Konfiguration des Inkontinenzartikels unterstützt wird.

Die für einen Inkontinenzartikel in Höschenform erforderliche elastische Dehnbarkeit in Quer- oder Hüftumfangsrichtung kann in vorteilhafter Weise dadurch realisiert werden, dass in dem Bauchabschnitt und/oder in dem Rückenabschnitt erste Elastifizierungsmittel vorgesehen sind, die sich in einem Abstand voneinander und im Wesentlichen in Quer- oder Hüftumfangsrichtung erstrecken und so den Bauchabschnitt und/oder den Rückenabschnitt flächenhaft elastifizieren, oder dass der Bauchabschnitt und/oder der Rückenabschnitt in Quer- oder Hüftumfangsrichtung elastisch dehnbare Flächenmaterialien umfasst. Insbesondere kann vorgesehen sein, dass der Bauchabschnitt und/oder der Rückenabschnitt zumindest außerhalb des Absorptionskörpers über die Längsrichtung im Wesentlichen durchgehend flächenhaft querelastifiziert sind. Eine Querelastifizierung ist also vom Grundsatz her dadurch möglich, dass im Wesentlichen fadenförmige Elastifizierungsmittel eingesetzt werden oder dass streifenförmige Elastifizierungsmittel oder alternativ auch zumindest in Querrichtung elastisch dehnbare Flächenmaterialien eingesetzt werden. In jedem Fall erweist es sich als vorteilhaft, wenn elastisch dehnbare Materialien mit im Wesentlichen undehnbaren Materialien, wie insbesondere Vliesmaterialien, kombiniert werden.

Es erweist sich insbesondere als vorteilhaft, wenn der Anteil der Fläche des Schrittabschnitts an der gesamten Fläche des Inkontinenzartikels 25 - 55 % beträgt. Insbesondere überlappt der Schrittabschnitt wenigstens 12 %, insbesondere 12% - 45%, weiter insbesondere 15% - 35%, weiter insbesondere 15% - 30% der Fläche des Bauchabschnitts. Insbesondere überlappt der Schrittabschnitt wenigstens 10 %, insbesondere 10% - 40%, weiter insbesondere 15% - 30%, weiter insbesondere 15% - 25% der Fläche des Rückenabschnitts.

Weiter vorteilhaft ist es, wenn der Anteil der flächenhaften Überlappung des Schrittabschnitts mit dem Rückenabschnitt insbesondere kleiner ist als mit dem Bauchabschnitt. Eine im Bauchabschnitt verhältnismäßig größere Überlappung trägt positiv zur Verwendung als Männer-Inkontinenzartikel bei.

Die Gesamtlängserstreckung L3 des Inkontinenzartikels beträgt vorzugsweise 650 - 950 mm, weiter vorzugsweise 700 - 950 mm, weiter vorzugsweise 700 - 900 mm, weiter vorzugsweise 720 - 880 mm. Die Gesamtlängserstreckung L3 des Inkontinenzartikels in Höschenform ist die maximale Längserstreckung gemessen in Längsrichtung des Inkontinenzartikels von der Hüftöffnungskante des Bauchabschnitts bis zur Hüftöffnungskante des Rückenabschnitts, also im nicht an den Seitennähten verbundenen, plan ausgebreiteten Zustand.

Die Querstreckung des Inkontinenzartikels beträgt vorzugsweise 500 - 960 mm, gemessen als maximale Erstreckung in Querrichtung des Inkontinenzartikels.

Der Inkontinenzartikel weist eine Gesamtfläche vorzugsweise von 250.000 - 500.000 mm², weiter vorzugsweise 250.000 - 450.000 mm² auf.

Insbesondere beträgt eine Fläche des Schrittabschnitts im eben ausgebreiteten Zustand 120.000 - 180.000 mm², weiter insbesondere 130.000 - 170.000 mm².

Der Schrittabschnitt weist vorzugsweise eine Längserstreckung von 500 - 800 mm, insbesondere von 500 - 700 mm auf. Eine maximale Erstreckung des Schrittabschnitts in Querrichtung beträgt vorzugsweise 160 - 300 mm, insbesondere 200 - 300 mm, weiter insbesondere 220 - 270 mm.

Der Schrittabschnitt weist vorzugsweise gerade Längsseitenkanten auf.

Insbesondere beträgt eine Fläche des Absorptionskörpers im eben ausgebreiteten Zustand 30.000 - 65.000 mm², insbesondere 35.000 - 60.000 mm², weiter insbesondere 40.000 - 55.000 mm².

Der Absorptionskörper weist vorzugsweise eine Längserstreckung, also eine Gesamtlänge S3 von 280 - 400 mm, weiter insbesondere von 300 - 380 mm, weiter insbesondere 300 - 370 mm auf.

Der Absorptionskörper weist vorzugsweise eine Quererstreckung, also Breite, vorzugsweise eine maximale Quererstreckung von 150 - 210 mm, insbesondere von 160 - 200 mm, weiter insbesondere 170 -190 mm auf. Insbesondere ist eine konturierte Ausbildung des Absorptionskörpers vorgesehen, vorzugsweise mit einer in einem vorderen Endbereich des Absorptionskörpers größeren Querstreckung als im hinteren Endbereich des Absorptionskörpers.

Insbesondere bevorzugt weist der Absorptionskörper an einer vorderen Produkthälfte des Inkontinenzartikels einen Flächenanteil von mindestens 15%, insbesondere 18 - 35%, weiter insbesondere 20 - 35% auf.

Insbesondere bevorzugt weist der Absorptionskörper an einer hinteren Produkthälfte des Inkontinenzartikels einen Flächenanteil von höchstens 10%, insbesondere höchstens 8%, weiter insbesondere höchstens 6ö, weiter insbesondere höchstens 4% auf, und weiter insbesondere mindestens 0,5%, weitere insbesondere mindestens 1%, weitere insbesondere mindestens 1,5% auf.

Weiter vorzugsweise ist der Absorptionskörper dergestalt, dass eine Fläche des Absorptionskörpers in einer hinteren Produkthälfte des Inkontinenzartikels (also hinter der Quermittelachse in Richtung Rückenabschnitt) einen Quotienten von einer maximalen Breite des Absorptionskörpers in der hinteren Produkthälfte zu einer maximalen Länge des Absorptionskörpers in der hinteren Produkthälfte (entspricht S2) von mindestens 1,0, weiter vorzugsweise mindestens 1,5, weitere vorzugsweise mindestens 1,7, weiter vorzugsweise mindestens 2,0, und weiter vorzugsweise höchstens 7,0, weiter vorzugsweise höchstens 6,0, weiter vorzugsweise höchstens 5,0 aufweist.

An dieser Stelle sei ausgeführt, dass in der vorliegenden Anmeldung jegliche Bemessungsangaben hinsichtlich Quererstreckung, Längserstreckung oder Abstand von Abschnitten oder an von an sich beliebigen Komponenten des Artikels im eben ausgebreiteten Zustand der den Artikel bildenden Flachmaterialien bestimmt werden, also gegebenenfalls nach Auftrennung der herstellerseitig schon angebrachten Seitennähte, so dass der betreffende Artikel in die in den Figuren dargestellte ebene Konfiguration gebracht werden kann.

Wenn der Artikel beispielsweise durch fadenförmige Elastifizierungsmittel im sogenannten "Stretchbondverfahren" elastifiziert wurde, so werden die Flächenmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen, Abmessungen oder Abstände ermittelt. Dieser Zustand ergibt sich bei undehnbaren Chassismaterialien auf Vliesbasis oder Vlies/Folien-Verbundbasis auf natürliche Weise wie in den Figuren dargestellt.

Bei dem hier in Rede stehenden dreiteiligen Aufbau des Inkontinenzartikels mit Bauchabschnitt, Rückenabschnitt und Schrittabschnitt erweist es sich insbesondere als vorteilhaft, wenn der Schrittabschnitt gewissermaßen als unitäre Einheit, vorzugsweise mit eingebrachten und fixierten Cuffelementen und vorzugsweise einschließlich der Schrittelastifizierungsmittel ausgebildet und als solche bezüglich des Bauchabschnitts und des Rückenabschnitts in Überlappung positioniert und fixiert wird. Insbesondere umfasst der Schrittabschnitt ein flüssigkeitsundurchlässiges Backsheet-Material und ein flüssigkeitsdurchlässiges Topsheet-Material, zwischen denen der Absorptionskörper angeordnet ist. Hierbei können das Backsheet-Material und/oder das Topsheet-Material in Querrichtung einen Überhang über den Absorptionskörper bilden. Solchenfalls erweist es sich als vorteilhaft, wenn die Schrittelastifizierungsmittel zumindest mit dem Topsheet-Material und/oder mit dem Backsheet-Material oder mit einem in Querrichtung nach außen verlaufendem Bereich des Materialabschnitts, welcher das Cuffelement bildet, verbunden sind.

Die Cuffelemente umfassen vorzugsweise ein Vliesmaterial, insbesondere ein hydrophobes Vliesmaterial, oder sind daraus gebildet.

Das Flächengewicht des hierfür eingesetzten Vliesmaterials für die Cuffelemente beträgt vorzugsweise 6-20 g/m², insbesondere 6-15 g/m², weiter insbesondere 8-15 g/m², weiter insbesondere 10-15 g/m².

Neben der Fixierung über die Cuffsockellinien kann vorgesehen sein, dass die Cuffelemente sich in Querrichtung distal über die Cuffsockellinie hinaus erstrecken und entlang einer vorzugsweise gerade verlaufenden, insbesondere parallel zur Längsmittelachse verlaufenden Cuffbasislinie am Schrittabschnitt festgelegt sind, wobei sich die Cuffbasislinie insbesondere über die gesamte Länge der Cuffelemente erstreckt, wobei die Cuffbasislinie distal zur Cuffsockellinie verläuft und/oder mit der Cuffsockellinie insbesondere abschnittweise zusammenfällt. Insbesondere können sich die Cuffelemente auch noch nach distal außerhalb der Cuffbasislinien erstrecken.

Vorzugsweise kann vorgesehen sein, dass sich die Cuffelemente über den Absorptionskörper hinaus im Schrittabschnitt in Richtung des Rückenabschnitts erstrecken und insbesondere über den gesamten Schrittabschnitt in Längsrichtung ausgebildet sind. Eine solche Fortführung der Cuffelemente ist vorteilhaft für die Stabilisierung des hinteren Schrittabschnitts, insbesondere aufgrund des nur geringen Anteils des Absorptionskörpers in diesem Bereich.

Weiterhin ist insbesondere vorgesehen, dass die Cuffelemente im hinteren Schrittabschnitt mit einem Material des Schrittabschnitts, insbesondere nur mit einer oberen körperzugewandten Lage des Schrittabschnitts, insbesondere einem Topsheet-Material nur bereichsweise, insbesondere über punktförmige Verbindungen, insbesondere über Ultraschallschweißstellen verbunden sind. Mit einer nur partiellen Fixierung kann neben der Fixierung der Materialen zueinander dennoch vorteilhaft eine weiches Anfühlungsvermögen der körperzugewandten Seite des Schrittabschnitts erhalten bleiben.

Gegenstand der Erfindung ist auch eine Anordnung (Array, Sortiment) aus einem ersten und einem zweiten Inkontinenzartikel, die einander im Hinblick auf verschiedene Benutzungssituationen in vorteilhafter Weise ergänzen und die sich in einer oder in mehrfacher Hinsicht voneinander unterscheiden.

Die Anordnung (Array, Sortiment) ergibt sich dabei durch die sinnfällige Herrichtung der zum Array gehörenden Inkontinenzartikel. Insbesondere durch die Beziehung oder das Verhältnis der Artikel zueinander. Diese erfolgt entweder bevorzugt durch Darbietung in einer gemeinsamen Verpackungseinheit und/oder durch Darbietung des ersten Inkontinenzartikels in einer Sortiment zugehörenden Verpackung und des zweiten Inkontinenzartikels in einer Sortiment zugehörenden Verpackung und/oder bevorzugt durch die Anbringung von Kennzeichnungen auf dem Inkontinenzartikel und/oder deren Verpackung und/oder die Darbietung in räumlicher oder inhaltlicher Nähe zueinander, die die Zugehörigkeit zu einem Array kenntlich machen. Die den Array bildenden Artikel stammen bevorzugt von ein- und demselben Hersteller.

Die einen Array bildenden Inkontinenzartikel weisen bevorzugt dieselbe Produktkennzeichnung auf, wie z. B. Markennamen und/oder Submarkennamen. Eine Anordnung aus einem ersten Inkontinenzartikel und einem zweiten Inkontinenzartikel wird verstanden als zumindest jeweils einem Vertreter davon und schließt eine Mehrzahl des einen ersten und/oder des einen zweiten Inkontinenzartikels ein. Erfindungsgemäß ist auch eine Anordnung von Inkontinenzartikeln für Männer in Höschenform, wobei die Anordnung umfasst: Einen ersten Inkontinenzartikel mit einer Längsmittelachse und einer ersten Quermittelachse für die Aufnahme von Körperausscheidungen, wobei der erste Inkontinenzartikel einen in Längsrichtung voneinander beabstandeten vorderen Bauchabschnitt und einen hinteren Rückenabschnitt, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, umfasst, und einen einen ersten Absorptionskörper aufweisenden Schrittabschnitt, der sich in einer Längsrichtung zwischen Bauchabschnitt und Rückenabschnitt erstreckt und an den Bauchabschnitt und den Rückenabschnitt in einem jeweiligen Überlappungsbereich unlösbar angefügt ist, und wobei der Absorptionskörper so angeordnet ist, dass eine erste Quermittellinie des ersten Absorptionskörpers zwischen der Quermittelachse des ersten Inkontinenzartikels und einer die Hüftöffnung begrenzenden Hüftöffnungskante des Bauchabschnitts angeordnet ist, wobei der Schrittabschnitt, der Bauchabschnitt und der Rückenabschnitt gemeinsam Beinöffnungen des Inkontinenzartikels begrenzen und mit eine seitliche Auslaufsperre bildenden und beidseits entlang der Längserstreckung des Absorptionskörpers verlaufenden Cuffelementen, die zumindest entlang einer Cuffsockellinie an einer körperzugewandten Seite des Inkontinenzartikels festgelegt sind und einen ersten unbefestigten freien zumindest abschnittsweise in der Längsrichtung elastifizierten Längsrand aufweisen, mit dem sie sich von der körperzugewandten Seite des Inkontinenzartikels emporerheben können und so die jeweilige seitliche Auslaufsperre bilden, wobei die Cuffelemente mit variierendem Abstand der Cuffsockellinien voneinander geführt sind und einen zweiten Inkontinenzartikel mit einer Längsmittelachse und einer zweiten Quermittelachse für die Aufnahme von Körperausscheidungen, wobei der zweite Inkontinenzartikel einen in Längsrichtung voneinander beabstandeten vorderen Bauchabschnitt und einen hinteren Rückenabschnitt, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, umfasst, und einen einen zweiten Absorptionskörper aufweisenden Schrittabschnitt, der sich in einer Längsrichtung zwischen Bauchabschnitt und Rückenabschnitt erstreckt und an den Bauchabschnitt und den Rückenabschnitt in einem jeweiligen Überlappungsbereich unlösbar angefügt ist, wobei der Schrittabschnitt, der Bauchabschnitt und der Rückenabschnitt gemeinsam Beinöffnungen des Inkontinenzartikels begrenzen und mit eine seitliche Auslaufsperre bildenden und beidseits entlang der Längserstreckung des Absorptionskörpers verlaufenden Cuffelementen, die zumindest entlang einer Cuffsockellinie an einer körperzugewandten Seite des zweiten Inkontinenzartikels festgelegt sind und einen zweiten unbefestigten freien zumindest abschnittsweise in der Längsrichtung elastifizierten Längsrand aufweisen, mit dem sie sich von der körperzugewandten Seite des Inkontinenzartikels emporerheben können und so die jeweilige seitliche Auslaufsperre bilden, und wobei eine Fläche des ersten Absorptionskörpers hinter der ersten Quermittelachse des ersten Inkontinenzartikels in Richtung auf den Rückenabschnitt des Inkontinenzartikels im ersten Inkontinenzartikel kleiner ist als eine Fläche des zweiten Absorptionskörpers hinter der zweiten Quermittelachse des zweiten Inkontinenzartikels in Richtung auf den Rückenabschnitt des zweiten Inkontinenzartikels.

Erfindungsgemäß handelt es sich bei dem ersten Inkontinenzartikel um einen vorstehend beschriebenen Inkontinenzartikel.

Insbesondere ist vorgesehen, dass der Abstand der Cuffsockellinien des ersten Inkontinenzartikels in Längsrichtung variiert gegenüber einem parallelen Verlauf der Cuffsockellinien des zweiten Inkontinenzartikels. Hierbei wird vorzugsweise der Bereich betrachtet, der zwischen den optional bevorzugt vorhandenen Cuffsockellinienendabschnitten liegt.

Insbesondere bevorzugt werden innerhalb eines Arrays ein erster und zweiter Inkontinenzartikel von im Wesentlichen gleicher Größe herangezogen. Die Größe eines Inkontinenzartikels zeigt sich insbesondere in der Länge und/oder in der Breite des Inkontinenzartikels (jeweils in plan ausgebreiteter Konfiguration, in einem nicht an den Seitennähten verbundenen Zustand). Als eine im Wesentlichen gleiche Größe wird hierin verstanden, dass der erste Inkontinenzartikel und der zweite Inkontinenzartikel überstimmen in der Länge und/oder in der Breite, insbesondere zumindest in der Breite übereinstimmen. Als eine im Wesentlichen gleiche Größe wird hierin insbesondere verstanden, dass eine Abweichung der Breite und/oder der Länge des ersten und zweiten Inkontinenzartikels voneinander höchstens 20%, weiter insbesondere höchstens 15%, weiter insbesondere höchstens 10%, weiter insbesondere höchstens 8% beträgt.

Vorzugsweise ist vorgesehen, dass die Fläche des ersten Absorptionskörpers hinter der ersten Quermittelachse des ersten Inkontinenzartikels um einen Faktor 2 - 9, vorzugsweise 3 - 9, weiter vorzugsweise 4 - 9, weiter vorzugsweise 4 - 8 kleiner ist als die Fläche des zweiten Absorptionskörpers hinter der zweiten Quermittelachse des zweiten Inkontinenzartikels.

Bevorzugt ist ebenfalls eine Gestaltung der Anordnung, bei der ein Verhältnis einer maximalen Höhe der Cuffelemente ausgehend von der Cuffsockellinie zum freien Längsrand zu einer Breite des ersten Absorptionskörpers gemessen auf der ersten Quermittelachse beim ersten Inkontinenzartikel um einen Faktor 1,05 - 1,50, insbesondere 1,10 - 1,45, weiter insbesondere 1,15 - 1,40 größer ist als das Verhältnis einer maximalen Höhe der Cuffelemente ausgehend von der Cuffsockellinie zum freien Längsrand zu einer Breite des zweiten Absorptionskörpers gemessen auf der zweiten Quermittelachse beim zweiten Inkontinenzartikel.

Vorzugsweise ist ein Verhältnis einer Längserstreckung C4 des unbefestigten freien Längsrandes hinter der Quermittelachse zu einer Längserstreckung C2 des freien Längsrandes vor der Quermittelachse, also C4/C2 des ersten Inkontinenzartikels um einen Faktor von mindestens 3, weiter vorzugsweise mindestens 3,5, weiter vorzugsweise mindestens 4, weitere vorzugsweise höchstens 6, vorzugsweise höchstens 5,5 kleiner als das Verhältnis C4 zu C2, C4/C2, des zweiten Inkontinenzartikels.

Insbesondere kann vorgesehen sein, dass ein Verhältnis S2/S1 aus einer Erstreckung S2 des ersten Absorptionskörpers hinter der ersten Quermittelachse zu einer Erstreckung S1 des ersten Absorptionskörpers vor der ersten Quermittelachse des ersten Inkontinenzartikels zum Verhältnis S2/S1 einer Erstreckung S2 des zweiten Absorptionskörpers hinter der zweiten Quermittelachse zu einer Erstreckung S1 des zweiten Absorptionskörpers vor der zweiten Quermittelachse des zweiten Inkontinenzartikels kleiner ist, insbesondere um den Faktor von mindestens 2, vorzugsweise mindestens 3, weiter vorzugweise höchstens 6, weiter vorzugsweise höchstens 5.

Die nachfolgenden Merkmalsaufzählungen können sich in beliebiger Kombination mit vorstehend erläuterten Merkmalen eines Inkontinenzartikels, insbesondere für einen ersten und/oder für einen zweiten Inkontinenzartikel als vorteilhaft erweisen:
- dass der Bauchabschnitt und/oder der Rückenabschnitt vorzugsweise eine von der Quer- oder Hüftumfangsrichtung abweichende und in Richtung auf eine Quermittelachse des Schrittabschnitts zulaufende Randkontur zur Begrenzung der Beinöffnungen aufweisen.
- dass in einem schrittseitigen und den Beinöffnungen zugewandten Bereich des Bauchabschnitts und des Rückenabschnitts vorzugsweise zweite Elastifizierungsmittel vorgesehen sind, die sich ausgehend von den beiden Seitennahtbereichen in Richtung auf eine Längsmittelachse des Inkontinenzartikels erstrecken und dabei bogenförmig sich auffächernd mit zunehmendem Abstand voneinander verlaufen.
- dass ein minimaler Abstand der zweiten Elastifizierungsmittel voneinander in den Seitennahtbereichen vorzugsweise 3 - 8 mm, insbesondere 3 - 7 mm beträgt.
- dass ein maximaler Abstand der zweiten Elastifizierungsmittel voneinander an einem Absorptionskörperrand oder an einem Längsrand des Schrittabschnitts vorzugsweise 10 - 35 mm beträgt.
- dass der maximale Abstand der zweiten Elastifizierungsmittel voneinander an einem Absorptionskörperrand oder an einem Längsrand des Schrittabschnitts im Rückenabschnitt vorzugsweise größer ist als im Bauchabschnitt.
- dass die ersten und/oder zweiten Elastifizierungsmittel vorzugsweise in einem Überlappungsbereich mit dem Absorptionskörper des Schrittabschnitts hinsichtlich ihrer elastischen Eigenschaften deaktiviert sind.
- dass der Abstand des schrittzugewandten innersten zweiten Elastifizierungsmittels des Bauchabschnitts von dem entsprechenden schrittzugewandten innersten zweiten Elastifizierungsmittel des Rückenabschnitts vorzugsweise 220 - 420 mm, insbesondere 220 - 400 mm beträgt.
- dass der Abstand der innersten, schrittzugewandten zweiten Elastifizierungsmittel von der die Beinöffnungen begrenzenden Randkontur des schrittseitigen und den Beinöffnungen zugewandten Bereichs des Bauchabschnitts und des Rückenabschnitts vorzugsweise 2 - 40 mm, vorzugsweise 3 - 30 mm, insbesondere vorzugsweise 4 - 15 mm beträgt.
- dass die Erstreckung des Bauchabschnitts und des Rückenabschnitts im Seitennahtbereich in Längsrichtung vorzugsweise wenigstens 100 mm, insbesondere wenigstens 120 mm und insbesondere 120 mm - 220 mm beträgt.

- dass der minimale Abstand des Bauchabschnitts und des Rückenabschnitts in Längsrichtung voneinander vorzugsweise 200 - 450 mm, insbesondere 200 - 420 mm beträgt.
- dass der Schrittabschnitt vorzugsweise ein flüssigkeitsundurchlässiges Backsheet-Material und ein Topsheet-Material umfasst, zwischen denen der Absorptionskörper angeordnet ist, wobei das Backsheet-Material und/oder das Topsheet-Material in Querrichtung einen Überhang über den Absorptionskörper bilden können und dieser Überhang
- in der Summe beidseits des Absorptionskörpers, also in der Summe links und rechts - vorzugsweise wenigstens 18 %, insbesondere 20 - 35 % und weiter insbesondere 25 - 32 % bezogen auf die größte Breite des Schrittabschnitts beträgt.
- dass die Schrittelastifizierungsmittel in Längsrichtung vorzugsweise vor den ersten und/oder zweiten Elastifizierungsmitteln enden.
- dass die Schrittelastifizierungsmittel in Längsrichtung vorzugsweise den Bauchabschnitt und/oder den Rückenabschnitt überlappen.
- dass der Bauchabschnitt und/oder der Rückenabschnitt zumindest außerhalb des Absorptionskörpers über die Längsrichtung vorzugsweise im Wesentlichen durchgehend flächenhaft querelastifiziert sind.

Weitere Einzelheiten, Merkmale und Vorteile der Erfindung ergeben sich aus den beigefügten Patentansprüchen und aus der zeichnerischen Darstellung und nachfolgenden Beschreibung bevorzugter Ausführungsformen des erfindungsgemäßen Inkontinenzartikels.

In der Zeichnung zeigen:
- Figur 1: einen Inkontinenzartikel gemäß der Erfindung in ausgebreiteter Konfiguration;
- Figur 2: eine Darstellung mit eingezeichneten Abmessungen;
- Figur 3: einen Ausschnitt des Inkontinenzartikels zur Darstellung der Bestimmung der mittleren vorderen und hinteren Cuffhöhe;
- Figur 4: einen Ausschnitt des Inkontinenzartikels zur Darstellung der Bestimmung des mittleren vorderen und hinteren Cuffsockellinienabstands;
- Figur 5: einen Ausschnitt des Inkontinenzartikels zur Darstellung der Bestimmung der mittleren vorderen und hinteren Absorptionskörperbreite;
- Figur 6a: einen Ausschnitt des Inkontinenzartikels mit in den Figuren 6b - 6f dazugehörigen Schnittdarstellungen;
- Figur 7: einen Schnitt durch einen Inkontinenzartikel gemäß Figur 1;
- Figur 8: einen Inkontinenzartikel in ausgebreiteter Konfiguration in einer weiteren Ausführungsform und
- Figur 9: einen Array gemäß der Erfindung.

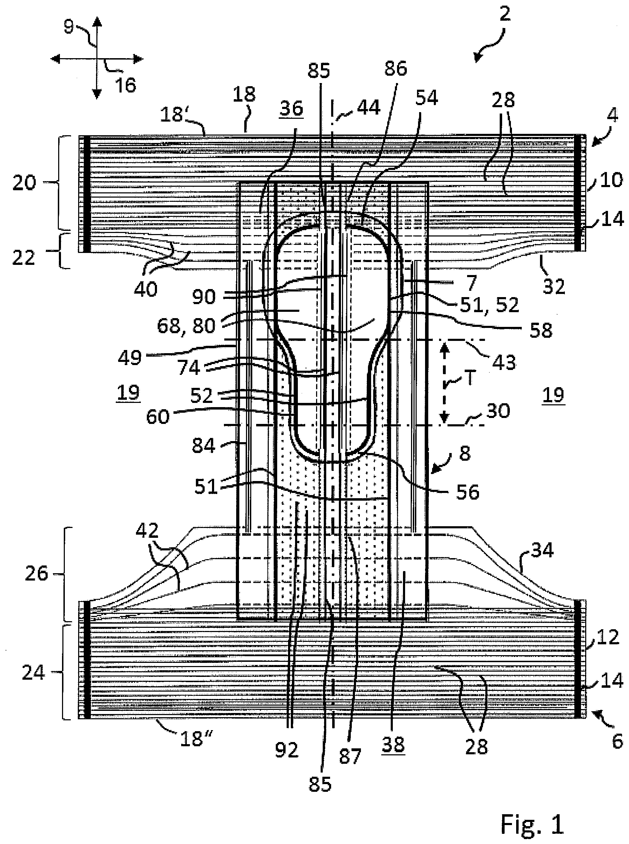

Die Figuren zeigen einen insgesamt mit dem Bezugszeichen 2 versehenen Inkontinenzartikel in Höschenform für die Aufnahme flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 sowie einem dazwischen angeordneten und einen Absorptionskörper 7 aufweisenden Schrittabschnitt 8, gebildet, wobei der Schrittabschnitt 8 mit einem Flächenanteil des Bauchabschnitts 4 einerseits und einem Flächenanteil des Rückenabschnitts 6 andererseits überlappt und im jeweiligen Überlappungsbereich 36, 38 herstellerseitig unlösbar verbunden ist. Wie aus Figur 1 ersichtlich, führt dies zu einer H-förmigen Grundstruktur des Inkontinenzartikels mit einer Längsrichtung 9. Die in Figur 1 dargestellten, miteinander gefügten Bestandteile werden dann zur Bildung der Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 ausgebildet werden. In diesem herstellerseitig gefertigten höschenförmigen Zustand des Inkontinenzartikels 2 erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 mit einer vorderen Hüftöffnungskante 18' und einer hinteren Hüftöffnungskante 18'' und Beinöffnungen 19, durch welche hindurch der Benutzer den Inkontinenzartikel 2 wie ein Höschen anlegt.

Der Bauchabschnitt 4 lässt sich bei der beispielhaften Ausführungsform in einen hüftseitigen Bereich 20 und einen beinöffnungsseitigen Bereich 22 unterteilen. Eine entsprechende Unterteilung ist im Rückenabschnitt 6 vorgesehen und zwar ebenfalls in einem hüftseitigen Bereich 24 und einem beinöffnungsseitigen Bereich 26.

In dem hüftseitigen Bereich 20 des Bauchabschnitts 4 und dem hüftseitigen Bereich 24 des Rückenabschnitts 6 sind erste Elastifizierungsmittel 28 vorgesehen, bei denen es sich insbesondere um fadenförmige Elastifizierungsmittel, wie Lycra^{®}-Fäden, handeln kann, die im vorgedehnten Zustand, im sogenannten "Stretchbondverfahren", mit den Flachmaterialien (Chassismaterialien) des Bauchabschnitts 4 und des Rückenabschnitts 6 verbunden sind. Diese ersten Elastifizierungsmittel 28 erstrecken sich in Quer- und/oder Hüftumfangsrichtung 16 von einem Seitennahtbereich 14 zum anderen.

Dabei kann vorgesehen sein, dass in der Nähe der Hüftöffnung 18 die Anzahl der elastischen Elemente 28 erhöht sein kann, um einen besonders guten Sitz im Hüftbereich sicherzustellen.

Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Inkontinenzartikels zulaufende Randkontur 32 bzw. 34. Diese Randkontur 32, 34 ist abschnittsweise bogenförmig und daher zur Begrenzung der Beinöffnungen 19 geeignet. Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist. Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus einer bevorzugten Ausführungsform in den Figuren zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt.

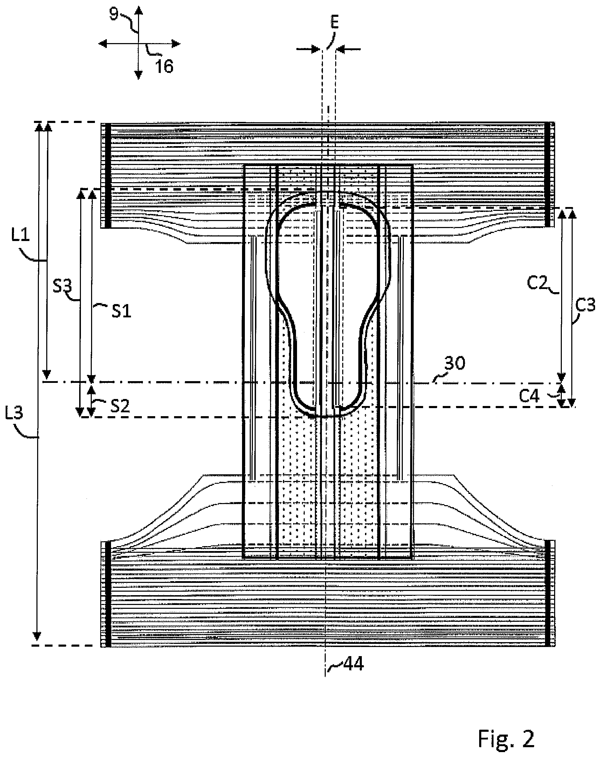

Darüber hinaus ist vorgesehen, dass der Absorptionskörper 7 in Richtung auf die Hüftöffnungskante 18' des Bauchabschnitts 8 nach vorne verschoben ist, so dass seine Quermittellinie 43, die sich ebenfalls aus der Halbierung der Länge des Absorptionskörpers 7 ergibt, gegenüber der Quermittelachse 30 des Inkontinenzartikels in Richtung auf den Bauchbereich um eine Länge T verschoben ist. Durch diese Vorverlagerung des Absorptionskörpers 7 in Richtung auf den Bauchabschnitt wird der männlichen Anatomie und dem Ort des Flüssigkeitsanfalls bei einem männlichen Benutzer verstärkt Rechnung getragen. Gleichwohl erstreckt sich der Absorptionskörper 7 mit einer Länge S2 (siehe Figur 2) auch in den rückseitigen Schrittbereich, um Leckagen zu vermeiden.

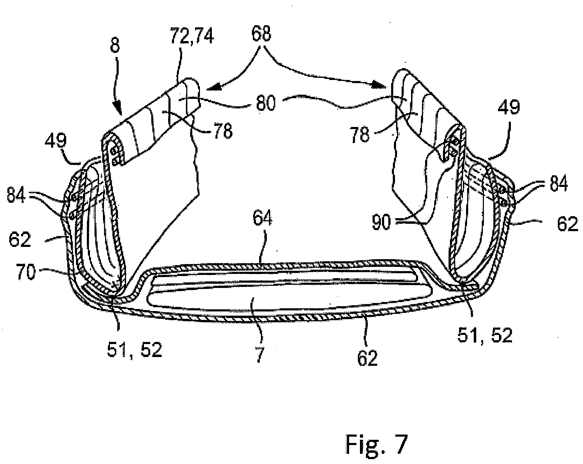

Wie auch in Figur 7 schematisch gezeigt, sind entlang des Absorptionskörpers 7 beidseits in Längsrichtung 9 verlaufend jeweils aufstehende Barrieremittel vorgesehen, welche als Cuffelemente 68 bezeichnet sind. Diese bestehen vorzugsweise aus einem hydrophoben, insbesondere flüssigkeitsundurchlässigen Vliesstoffmaterial. Das Cuffelement 68 wird dabei durch einen Vliesmaterialabschnitt 70 gebildet, der sich vorzugsweise über die gesamte Länge des Schrittabschnitts 8 erstreckt und bis an die Außenkanten 49 des Schrittabschnitts in Querrichtung 16 reicht. Die Cuffelemente 68 sind dabei entlang einer Cuffbasislinie 51 mit dem Schrittabschnitt 8 verbunden, wobei die Cuffbasislinie insbesondere durch eine Ultraschallverbindung den Kontakt hergestellt. Darüber hinaus weist das Cuffelement 68 eine Cuffsockellinie 52 auf, die bereichsweise mit der Cuffbasislinie 51 zusammenfällt. Die Cuffbasislinie 51 verläuft dabei vorzugsweise parallel zur Längsmittelachse 44 des Inkontinenzartikels, wohingegen die Cuffsockellinie 52 eine Konturierung aufweist und die Cuffsockellinie 52 des einen Cuffelements 68 einen variierenden Abstand zur Cuffsockellinie 52 des anderen Cuffelements 68 aufweist. Dabei folgt die Konturierung der Cuffsockellinie 52 im Wesentlichen der Form des Absorptionskörpers 7, der eine glühbirnenartige Form aufweist. Dabei ist die Cuffsockellinie 52 in einer vorteilhaften Ausführung in den beiden Endbereichen in Querrichtung 16 bogenförmig nach innen, also in Richtung auf einen unbefestigten freien Längsrand 74 der Cuffelemente 68, der vom Schrittbereich aufsteht, ausgebildet, der den emporerhebbaren Bereich 80 der Cuffelemente nach hinten und vorne begrenzt. Der Cuffsockellinienendabschnitt ist vorne mit dem Bezugszeichen 54 und hinten mit dem Bezugszeichen 56 bezeichnet. Auf diese Weise wird eine Art Tasche gebildet, die insbesondere vorteilhaft zur Aufnahme des männlichen Genitals dienen kann. Ausgehend von den Cuffsockellinienendabschnitten 54 und 56 sind die nach innen gerichteten Längsränder des Cuffelements 68, die mit dem Bezugszeichen 85 bezeichnet sind, über Cuffendenfixierungen 86 und 87 auf dem Material des Schrittabschnitts 8 festgelegt, wobei dies insbesondere über eine Ultraschallverbindung erfolgt.

In Querrichtung außerhalb und beidseits des Absorptionskörpers 7 sind in der Längsrichtung 9 verlaufende und der jeweiligen Beinöffnung 19 zugeordnete Schrittelastifizierungsmittel 84 vorgesehen. Sie sind im beispielhaft schematisch dargestellten Fall in Figur 7 in einem Querabschnitt zur Cuffbasislinie 51 und/oder Cuffsockellinie 52 zwischen dem Vliesmaterialabschnitt 70 der Cuffelemente 68 und dem Backsheetmaterial 62 angeordnet. Sie vermögen somit den Schrittabschnitt 8 in der Längsrichtung 9 zu elastifizieren und eine Raffung des Chassismaterials des Schrittabschnitts 8 herbeizuführen. In der Gebrauchssituation erheben sich daher die Längsrandbereiche 49 des Schrittabschnitts 8 gegen die Beine des Benutzers und bilden einen Seitenauslaufschutz des Inkontinenzartikels. Durch die hiermit verbundene Bootform des Schrittabschnitts 8 wird das Aufrichten der emporerhebbaren Bereiche 80 der jeweiligen Cuffelemente 68, was in Figur 7 schematisch dargestellt ist, weiter unterstützt. Dieser emporerhebbare Bereich 80 des Cuffelements 68 umfasst über einen bestimmten Bereich ein Elastifizierungsmittel 90, beispielsweise zwei fadenförmige Elastifizierungsmittel, wie Lycra^{®}-Fäden, die in dem umgefalteten Bereich 78 im vorgedehnten Zustand, im sogenannten Stretch-Bond-Verfahren, mit dem Material der Cuffelemente 68 verbunden worden sind. Die Elastifizierungsmittel 90 dienen dazu, das Aufstellen und Emporerheben der Cuffelemente 68 insbesondere bei breiten Absorptionskörpern 7, wie sie bei bevorzugt für Männer verwendbaren Produkten eingesetzt werden, zu verbessern. Das Cuffelement 68 ist aus einem auf sich selbst entlang der Längsrichtung 9 umgefalteten Vliesmaterialabschnitt 70 gebildet, wobei eine hierbei entstehende Faltlinie 72 einen unbefestigten freien Längsrand 74 bildet.

Wie in Figur 1 weiter schematisch dargestellt, so sind die Cuffelemente 68 im Bereich außerhalb des Absorptionskörpers 7 mit dem Material des Schrittabschnitts 8, hier insbesondere dem Topsheetmaterial, über punktförmige Klebe- oder Schweißverbindungen 92 verbunden, so dass der rückseitige Bereich des Inkontinenzartikels 2, der dem Rückenabschnitt 6 zugewandt ist, verstärkt wird. Die Verbindungspunkte 92 sind dabei vorzugsweise in Reihen angeordnet, die in Längsrichtung 9 verlaufen. Hierdurch wird die hintere Raffung durch die rückseitigen Elastifizierungsmittel 28 im Rückenabschnitt nicht gestört, bei gleichzeitiger Stabilisierung des rückseitigen Schrittabschnitts, der im vorliegenden Fall aufgrund der Kürze des Absorptionskörpers 7 nicht durch den Absorptionskörper stabilisiert und an einem Zusammenfallen gehindert wird.

Die Aufrichtung der Cuffelemente 68 wird weiterhin durch die Elastifizierung 90 der Cuffelemente, die in Längsrichtung 9 im Wesentlichen an dem freien Längsrand 74 der Cuffelemente 68 verläuft, bereitgestellt.

Mit der Konturierung der Cuffsockellinie 52 geht insbesondere einher, dass die Cuffhöhe in einem vorderen Bereich höher ist als an in einem hinteren Bereich, wodurch die Taschenbildung insbesondere im vorderen Bereich weiter unterstützt werden kann und die Sicherheit des Flüssigkeitsauffangs erhöht werden kann.

In einer bevorzugten Ausführungsform des Inkontinenzartikels zeigt sich die Ausgestaltung der Cuffhöhe innerhalb des Verlaufs des freien ungebundenen Längsrandes des Cuffelements in einem Verhältnis einer mittleren vorderen Cuffhöhe F(v) zu einer mittleren hinteren Cuffhöhe F(h), als Quotient F(v)/F(h) von mindestens 1,2 und insbesondere höchstens 2,0.

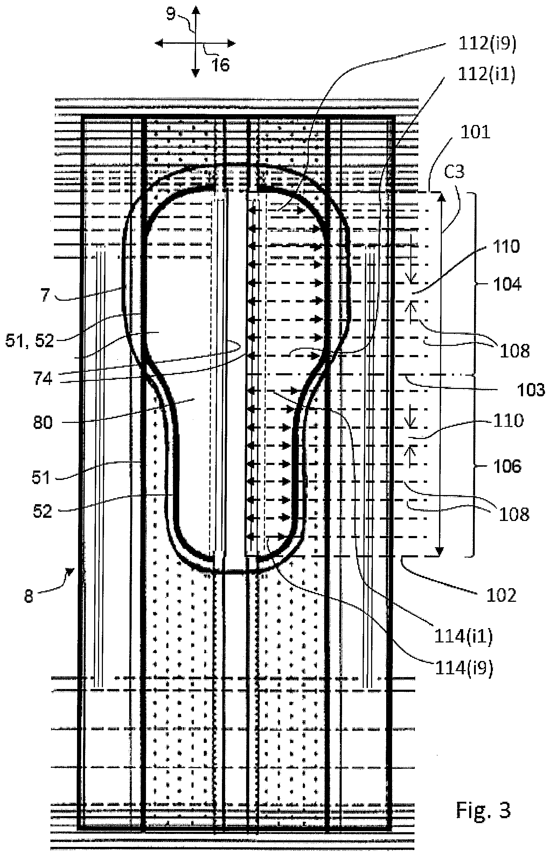

Zur Bestimmung der mittleren vorderen und mittleren hinteren Cuffhöhe wird, wie in Figur 3 schematisch dargestellt, das Cuffelement innerhalb des Verlaufs des freien ungebundenen Längsrandes 74 mit einer Gesamtlängserstreckung C3 zwischen zwei in Querrichtung 16 verlaufenden Hilfslinien 101 und 102 mittels einer ebenso in Querrichtung angelegten Halbierungslinie 103 in ein vorderes Längsrand-Cuffsegment 104 und ein hinteres Längsrand-Cuffsegment 106 halbiert. Das vordere Längsrand-Cuffsegment 104 und das hintere Längsrand-Cuffsegment 106 wird jeweils in der Längsrichtung 9 durch Anlegen von neun in Längsrichtung äquidistanten, also mit gleichem Abstand 110, und in Querrichtung verlaufenden imaginären Sektionstrennlinien 108 aufgeteilt, wobei die Sektionstrennlinien 108 den unbefestigten freien Längsrand 74 und die Cuffsockellinie 52 schneiden. Innerhalb des vorderen Längsrand-Cuffsegments 104 wird zwischen der Cuffsockellinie 52 und dem unbefestigten freien Längsrand 74 entlang jeder einzelnen der neun Sektionstrennlinien 108 der Abstand 112 (i1) bis 112 (i9) jeweils als einzelne Cuffhöhe F i(v) vermessen, analog dazu im hinteren Längrand-Cuffsegment 108 die Abstände 114 (i1) bis 114 (i9) als einzelne Cuffhöhe F i(h). Eine Summe der einzelnen Cuffhöhen im vorderen Längrand-Cuffsegment und die Summe der einzelnen Cuffhöhen in der hinteren Längsrand-Cuffsegment wird jeweils durch n= 9 geteilt, zum Erhalt der mittleren vorderen Cuffhöhe F(v) und der mittleren hinteren Cuffhöhe F(h). Daraus wird das Verhältnis der mittleren vorderen Cuffhöhe zur mittleren hinteren Cuffhöhe als Quotient F(v)/F(h) gebildet.

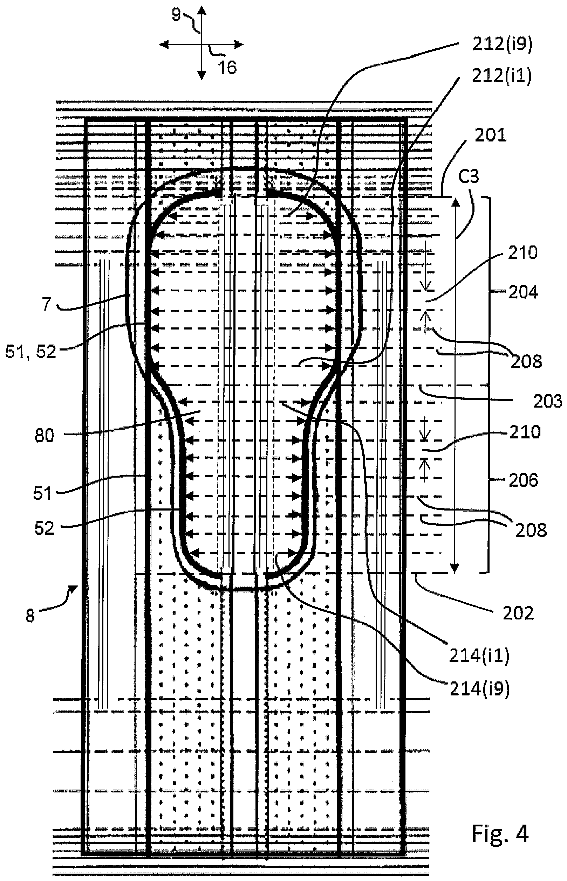

Für die Ermittlung des mittleren vorderen und mittleren hinteren Cuffsockellinienabstands wird, anhand von der Figur 4 erläutert, wie folgt verfahren: Mit einer Halbierung des über die Gesamtlängserstreckung C3 zwischen zwei in Querrichtung 16 verlaufenden Hilfslinien 201 und 202 und zwar innerhalb der beiden Cuffsockellinien 52 vorhandenen Bereichs mittels einer in Querrichtung angelegten Halbierungslinie 203 wird ein vorderes Cuffsockellinienabstandsegment 204 und in ein hinteres Cuffsockellinienabstandsegment 206 erhalten. Innerhalb des jeweiligen Segments 204, 206 werden auf neun in Längsrichtung 9 mit einer gleichen Distanz 210 und in Querrichtung 16 verlaufenden imaginären Sektionstrennlinien 208, die Distanz zwischen den beiden Cuffsockellinien vermessen, was jeweils einem einzelnen Cuffsockellinienabstand 212 von 212 (i1) bis 212 (i9) innerhalb des vorderen Cuffsockellinienabstandsegment 204 und jeweils einem einzelnen Cuffsockellinienabstand 214 von 214 (i1) bis 214 (i9) innerhalb des hinteren Cuffsockellinienabstandsegment 206 entspricht. Für das vordere 204 und das hintere Cuffsockellinienabstandsegment 206 wird aus der Summe der jeweils einzelnen Cuffsockellinienabstände und anschließender Division durch n=9 der mittlere vordere Cuffsockellinienabstand und der mittlere hintere Cuffsockellinienabstand ermittelt. Das Verhältnis vom mittleren vorderen Cuffsockellinienabstand zum mittleren hinteren Cuffsockellinienabstand wird als Quotient D(v)/D(h) berechnet.

In einer bevorzugten Ausführungsform beträgt das Verhältnis des mittleren vorderen Cuffsockellinienabstand zum mittleren hinteren Cuffsockellinienabstand, D(v)/D(h), mindestens 1,2, insbesondere mindestens 1,3, insbesondere mindestens 1,4, insbesondere mindestens 1,5 und insbesondere höchstens 2,0, weiter insbesondere höchstens 1,9, weiter insbesondere höchstens 1,8, weiter insbesondere höchstens 1,7.

Bei einer bevorzugten Ausgestaltung der Cuffsockellinien 52 sind jeweils ein vorderer sich parallel zur Längsmittelachse 44 erstreckender Längsabschnitt 58 und insbesondere auch ein hinterer sich parallel zur Längsmittelachse 44 erstreckender Längsabschnitt 60 vorgesehen.

In einer bevorzugten Ausführungsform des Inkontinenzartikels weist der Absorptionskörper 7 eine mittlere vordere Absorptionskörperbreite G(v) und eine mittlere hintere Absorptionskörperbreite G(h) in einem Verhältnis G(v)/G(h) von mindestens 1,2, insbesondere mindestens 1,3, insbesondere mindestens 1,4 und insbesondere höchstens 2,0, weiter insbesondere höchstens 1,9, weiter insbesondere höchstens 1,8 auf.

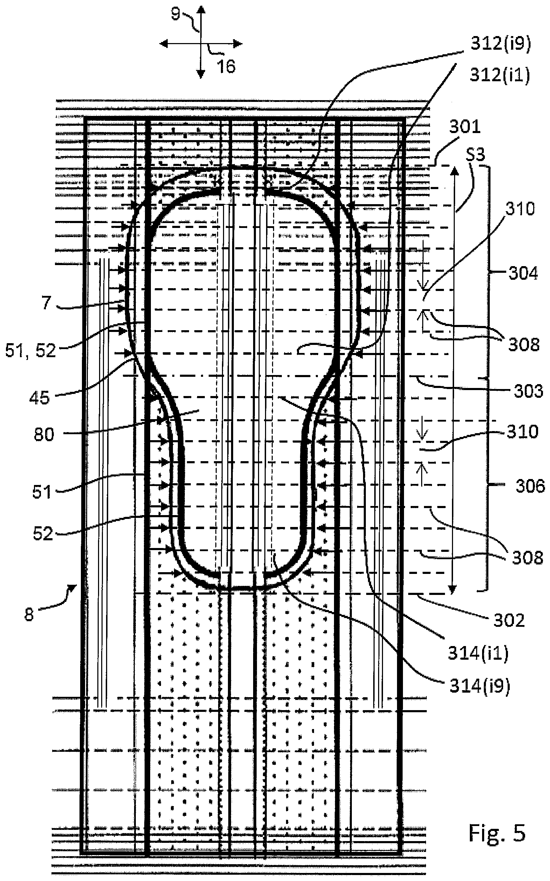

Für die Bestimmung dieses Verhältnisses wird, wie anhand Figur 5 erläutert, der Absorptionskörper 7 über seine Gesamtlängserstreckung S3 zwischen einer parallel am jeweiligen Längsende des Absorptionskörpers angeordneten in Querrichtung 16 verlaufende vorderen und hinteren Hilfslinie 301, 302 und der den Absorptionskörper umschreibenden Konturlinie 45 mittels einer Halbierungslinie 303 in ein vorderes Absorptionskörpersegment 304 und ein hinteres Absorptionskörpersegment 306 unterteilt. Entlang und auf jeweils neun in Längsrichtung 9 mit gleichem Abstand 310 zueinander und in Querrichtung 16 verlaufenden Sektionstrennlinien 312 innerhalb des vorderen Absorptionskörpersegments 304 und neun Sektionstrennlinien 314 innerhalb des hinteren Absorptionskörpersegment 306 wird zwischen der Absorptionskörperkontur 45 jeweils eine einzelne Absorptionskörperbreite von 312 (i1) bis 312(i9) und von 314(i1) bis 314 (i9) vermessen. Die Summe der einzelnen Absorptionskörperbreiten im jeweiligen vorderen und hinteren Absorptionskörpersegment und deren Division durch n=9 ergibt die mittlere vordere Absorptionskörperbreite G(v) und die mittlere hintere Absorptionskörperbreite G(h).

Vorzugsweise folgt die Cuffsockellinie 52 zumindest abschnittsweise der Kontur 45 des Absorptionskörpers 7. Vorzugsweise ist die Cuffsockellinie 52 innerhalb der Kontur 45 des Absorptionskörpers 7 angeordnet.

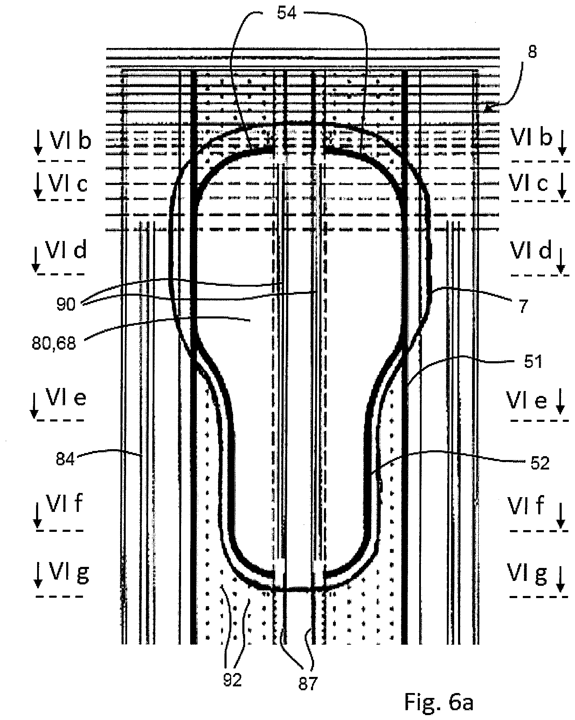

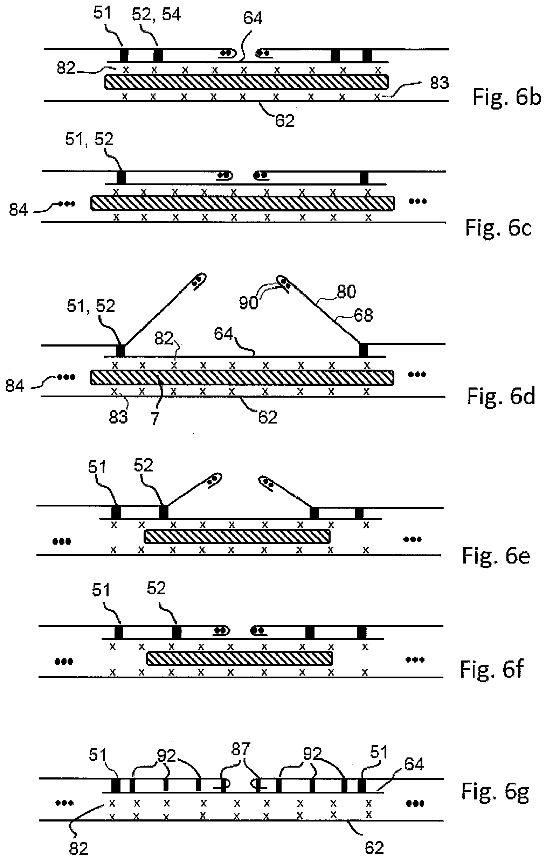

Die Figuren 6b - 6g zeigen schematisch Schnittdarstellungen an verschiedenen Positionen aus der Figur 6a, die schematisch einen Ausschnitt des Schrittabschnitts 8 mit einem Absorptionskörper 7 und mit beidseitigen Cuffelementen 68 zeigt. Beidseitig vom Absorptionskörper 7, der zwischen Backsheet-Material 62 und einem Topsheet-Material 64 und auch Cuffelementen 68 angeordnet und darin beispielsweise mittels Kleber 82, 83 fixiert ist, sind vorzugsweise Schrittelastifizierungsmittel 84 eingebracht. In den Schnittdarstellungen Figuren 6b- 6g wird die Führung der Cuffsockellinien 52 mit einem variierenden Abstand voneinander in Längsrichtung als auch die Führung der Längskanten des Absorptionskörpers voneinander in Längsrichtung, also eine variierende Absorptionskörperbreite verdeutlicht. Beginnend in einem vorderen Bereich, wie in Figur 6b dargestellt, so weisen die Cuffsockellinien 52 einen vorderen Cuffsockellinienendabschnitt 54 auf, der einen Abschluss des emporerhebbaren Bereichs 80 der Cuffelemente 68 miteinbringt. Das Aufstellen des emporerhebbaren Bereich 80 der Cuffelemente 68 wird durch eine eingebrachte Elastifizierung 90 der Cuffelemente unterstützt. Die Cuffsockellinie 52, als Verbindungstelle mit einer körperzugewandten Lage des Schrittabschnitts, wie dem Topsheet-Material 64 begrenzt den emporerhebbaren Bereich 80 der Cuffelemente proximal. Die Cuffelemente sind weiter vorzugsweise beidseitig vom Absorptionskörper mittels einer Cuffbasislinie 51, und vor allem in einem zum Rückenabschnitt orientierten Bereich im Schrittabschnitt weiter auf dem Topsheet-Material 64 fixiert. Zudem sind punktförmige Verbindungen 92, wie Klebe- und/oder Schweißstellen vorgesehen, die neben der Cuffendenfixierung 87 der nach innen gerichteten Längsränder der Cuffelemente für eine Stabilisierung des hinteren Schrittabschnitts sorgen und dennoch ein weiches Anfühlungsvermögen erhalten bleibt.

Figur 2 zeigt nun den Inkontinenzartikel gemäß Figur 1 im eben ausgebreiteten Zustand, wobei folgende Bemessungen vorgesehen wurden:
- Längserstreckung L3 des gesamten Inkontinenzartikels 2 sowie L1 Längserstreckung des halbierten Inkontinenzartikels
- Gesamtlängserstreckung C3 des unbefestigten freien Längsrandes der Cuffelemente und dessen Längserstreckung C2 ab der Quermittelachse 30 des Inkontinenzartikels in Richtung Bauchabschnitt sowie Längserstreckung C4 des unbefestigten freien Längsrandes ab der Quermittelachse 30 des Inkontinenzartikels in Richtung Rückenabschnitt
- Gesamtlänge Absorptionskörper S3 sowie Längserstreckung S1 des Absorptionskörpers ab der Quermittelachse 30 des Inkontinenzartikels in Richtung Bauschabschnitt und Längserstreckung S2 des Absorptionskörpers ab der Quermittelachse 30 des Inkontinenzartikels in Richtung Rückenabschnitt
- Abstand E zwischen den freien Kanten der Cuffelemente am vorderen fixierten Endrandabschnitt

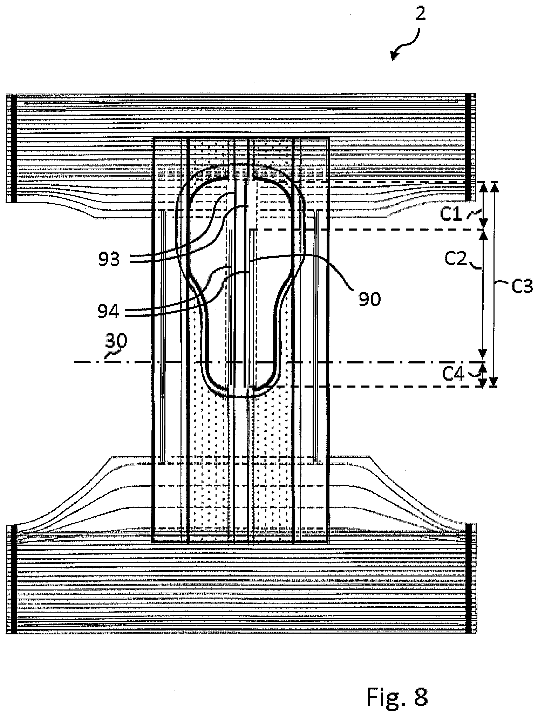

Figur 8 zeigt eine bevorzugte Ausführungsform des Inkontinenzartikels 2 mit einem nicht elastifizierten freien Längsrandendabschnitt 93 mit einer Längserstreckung C1, der in Richtung Bauchabschnitt vor einem mit Elastifizierungsmitteln 90 erhaltenen elastischen Abschnitt 94 des freien unbefestigten freien Längsrandes der Cuffelemente angeordnet ist.

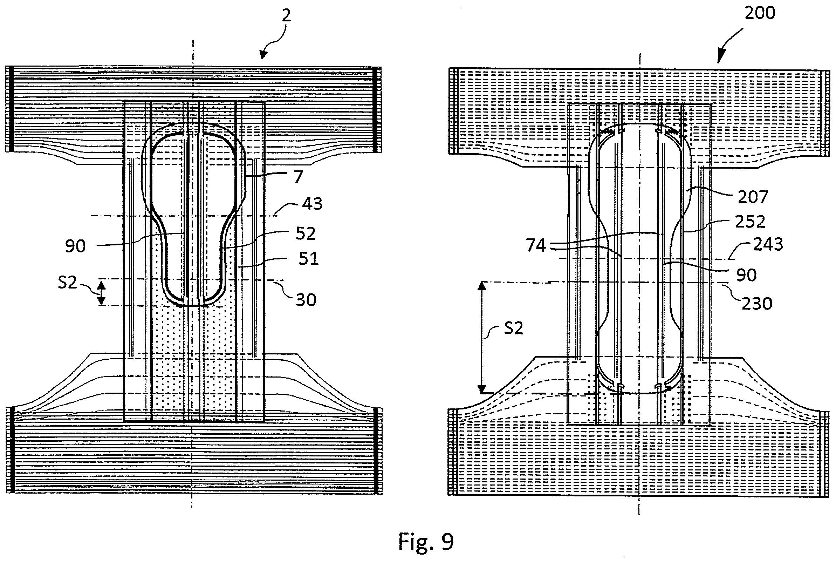

Schließlich zeigt Figur 9 eine Draufsicht auf einen weiteren zweiten Inkontinenzartikel 200, der sich von dem bislang beschriebenen und ebenfalls dargestellten Inkontinenzartikel 2, wobei auch der zweite Inkontinenzartikel 200 ein genderspezifischer Männerartikel ist, insbesondere dadurch unterscheidet, dass die erste Quermittellinie 43 des Absorptionskörpers 7 des ersten Inkontinenzartikel 2 noch weiter in Richtung auf die Hüftöffnungsvorderkante 18' des Bauchabschnitts nach vorne verschoben ist als die Quermittellinie 243 des zweiten Absorptionskörpers 207 des zweiten Inkontinenzartikels, da der Absorptionskörper 7 insgesamt kürzer ausgebildet ist und sich nur um das Maß S2 in Richtung der Rückseite über die Quermittelachse 30 des Inkontinenzartikels hinüber erstreckt. Die zweite Verbreiterung des weiteren zweiten Inkontinenzartikels 200, die der Absorptionskörper 207 zum Rückenabschnitt hin aufweist, ist beim erfindungsgemäßen Inkontinenzartikel 2 vollständig entfallen. Auf diese Weise kann ein Inkontinenzartikel 2 bereitgestellt werden, der insbesondere für die Verwendung als Tagprodukt von Männern vorgesehen sein kann, so dass insbesondere auf der Rückseite des Inkontinenzartikels 2 eine diskretere unterwäscheartigere Erscheinung möglich ist. Darüber hinaus sind die Cuffelemente beim Inkontinenzartikel 2 bezüglich ihrer Cuffsockellinien 52 konturiert ausgebildet, so dass die Cuffhöhe über die Länge der Cuffelemente variiert. Hingegen verlaufen die Cuffsockellinien 252 des zweiten Inkontinenzartikels 200 außer in den Endbereichen in ihrer Erstreckung gerade parallel.

Auf diese Weise kann beim Inkontinenzartikel 2 besonders gut eine Tasche gebildet werden, die das männliche Genital umschließt und so der Aufnahme von Flüssigkeiten zuträglich ist. Durch diese beiden Maßnahmen wird der Inkontinenzartikel 2 geschaffen, der insbesondere an die Bedürfnisse und anatomischen Gegebenheiten eines männlichen Trägers bei Tropfinkontinenz, leichter und/oder mittlerer Inkontinenz angepasst ist, im Vergleich zu einem Inkontinenzartikel 200, der als zweiter Inkontinenzartikel vorzugsweise an die Bedürfnisse eines Trägers mit stärkerer Inkontinenz bzw. einem weniger mobilen Träger angepasst ist.

Die unterschiedlichen Benutzungszwecke/Einsatzbereiche der beiden Inkontinenzartikel zeigt sich insbesondere auch darin, dass die Fläche des ersten Absorptionskörpers 7 hinter der ersten Quermittelachse 30 des Inkontinenzartikels beim ersten Inkontinenzartikel 2 um einen Faktor 2 - 9, vorzugsweise 3 - 9, weiter vorzugsweise 4 - 9, weiter vorzugsweise 4 - 8 kleiner ist als die Fläche des zweiten Absorptionskörpers 207 hinter der zweiten Quermittelachse 230 des zweiten Inkontinenzartikels 200.

In Betrachtung der Erstreckung des jeweiligen Absorptionskörpers ist das Verhältnis S2/S1 aus einer Erstreckung S2 des ersten Absorptionskörpers 7 hinter der ersten Quermittelachse 30 zu einer Erstreckung S1 des ersten Absorptionskörpers des ersten Inkontinenzartikels 2 vor der ersten Quermittelachse zum entsprechenden Verhältnis S2/S1 einer Erstreckung S2 des zweiten Absorptionskörpers 207 hinter der zweiten Quermittelachse 230 zu einer Erstreckung S1 des zweiten Absorptionskörpers vor der zweiten Quermittelachse 230 des zweiten Inkontinenzartikels 200 kleiner und dabei insbesondere um den Faktor von mindestens 2, vorzugsweise mindestens 3, weiter vorzugweise höchstens 6, weiter vorzugsweise höchstens 5.

Mit Blick auf die Cuffelemente und der Erstreckung des freien Längsrandes 74 ist ein Verhältnis einer Längserstreckung C4 des unbefestigten freien Längsrandes hinter der Quermittelachse zu einer Längserstreckung C2 des freien Längsrandes vor der Quermittelachse, also C4/C2 des ersten Inkontinenzartikels 2 um einen Faktor von mindestens 3, weiter vorzugsweise mindestens 3,5, weiter vorzugsweise mindestens 4, weitere vorzugsweise höchstens 6, vorzugsweise höchstens 5,5 kleiner als das Verhältnis C4 zu C2 des zweiten Inkontinenzartikels 200.

Die beiden Inkontinenzartikel zusammen bilden eine beanspruchte Anordnung ("Array").

## Patentansprüche

1. Wegwerfbarer Inkontinenzartikel (2) in Höschenform mit einer Längsmittelachse (44) und einer Quermittelachse (30) für die Aufnahme von Körperausscheidungen für die bevorzugte Verwendung durch Männer, mit einem in einer Längsrichtung (9) voneinander beabstandeten vorderen Bauchabschnitt (4) und einem hinteren Rückenabschnitt (6), die zur Bildung eines in Quer- oder Hüftumfangsrichtung (16) durchgehenden Bauch- und Rückenbands mit einer in Hüftumfangsrichtung (16) geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8), der sich in einer Längsrichtung (9) zwischen Bauchabschnitt (4) und Rückenabschnitt (16) erstreckt und an den Bauchabschnitt (4) und den Rückenabschnitt (6) in einem jeweiligen Überlappungsbereich (36, 38) unlösbar angefügt ist, wobei der Schrittabschnitt (8), der Bauchabschnitt (4) und der Rückenabschnitt (6) gemeinsam Beinöffnungen (19) des Inkontinenzartikels (2) begrenzen und wobei der Schrittabschnitt (8) beidseits eine seitliche Auslaufsperre bildende und beidseits entlang einer Längserstreckung des Absorptionskörpers (7) verlaufende Cuffelemente (68) aufweist, die zumindest entlang einer Cuffsockellinie (52) an einer körperzugewandten Seite des Inkontinenzartikels (2) festgelegt sind und einen unbefestigten freien Längsrand (74) aufweisen, der zumindest abschnittsweise in der Längsrichtung (9) elastifiziert ist, mit dem sich die Cuffelemente (68) von der körperzugewandten Seite des Inkontinenzartikels (2) emporerheben können und so die jeweilige seitliche Auslaufsperre bilden und wobei der Absorptionskörper (7) so angeordnet ist, dass eine Quermittellinie (43) des Absorptionskörpers (7) zwischen der Quermittelachse (30) des Inkontinenzartikels (2) und einer die Hüftöffnung (18) begrenzenden Hüftöffnungskante (18') des Bauchabschnitts (4) angeordnet ist, und wobei die Cuffelemente(68) mit variierendem Abstand der Cuffsockellinien (52) voneinander geführt sind, und wobei sich der Absorptionskörper (7) in Richtung des Rückenabschnitts (6) über die Quermittelachse (30) des Inkontinenzartikels in Richtung einer Hüftöffnungskante (18") des Rückenabschnitts (6) erstreckt, **dadurch gekennzeichnet, dass** ein Verhältnis einer Länge S2 des Absorptionskörpers (7) hinter der Quermittelachse (30) des Inkontinenzartikels (2) in Richtung Rückenabschnitt (6) zu einer Länge S1 des Absorptionskörpers (7) vor der Quermittelachse (30) des Inkontinenzartikels (2) in Richtung Bauchabschnitt (4) höchstens 0,30 beträgt.

2. Inkontinenzartikel nach Anspruch 1, **dadurch gekennzeichnet, dass** die jeweilige Cuffsockellinie (52) einen in Querrichtung (16), insbesondere bogenförmig, nach innen in Richtung auf eine Längsmittelachse (44) des Inkontinenzartikels (2) und damit in Richtung auf den freien Längsrand (74) des jeweiligen Cuffelements (68) zu verlaufenden vorderen Cuffsockellinienendabschnitt (54) und/oder hinteren Cuffsockellinienendabschnitt (56) aufweist, wobei dieser vordere und/oder hintere Cuffsockellinienendabschnitt (54,56) den emporerhebbaren Bereich (80) des Cuffelements (68) in der Längsrichtung (9) begrenzt.

3. Inkontinenzartikel nach einem der vorangehenden Ansprüche, **dadurch gekennzeichnet, dass** ein Verhältnis eines mittleren vorderen Cuffsockellinienabstands D(v) in einem vorderem Cuffsockellinienabstandsegment zu einem mittleren hinteren Cuffsockellinienabstand D(h) in einem hinteren Cuffsockellinienabstandsegment mindestens 1,2, insbesondere mindestens 1,3, insbesondere mindestens 1,4, und weiter insbesondere höchstens 2,3, weiter insbesondere höchstens 2,2, weiter insbesondere höchstens 2,1, weiter insbesondere höchstens 2,0, weiter insbesondere höchstens 1,9, weiter insbesondere höchstens 1,8, weiter insbesondere höchstens 1,7 beträgt.

4. Inkontinenzartikel nach einem der vorangehenden Ansprüche, **dadurch gekennzeichnet, dass** ein Verhältnis eines maximalen Abstands der Cuffsockellinien (52) innerhalb einer vorderen Produkthälfte vor der Quermittelachse des Inkontinenzartikels zu einem Abstand der Cuffsockellinien (52) vermessen auf der Quermittelachse (30) des Inkontinenzartikels mindestens 1,3, insbesondere mindestens 1,4, und insbesondere höchstens 2,0, weiter insbesondere höchstens 1,8 beträgt.

5. Inkontinenzartikel nach einem der vorangehenden Ansprüche, **dadurch gekennzeichnet, dass** ein Verhältnis einer Längserstreckung C4 des unbefestigten freien Längsrandes (74) hinter der Quermittelachse (30) des Inkontinenzartikels (2) in Richtung Rückenabschnitt (6) zu einer Längserstreckung C2 des unbefestigten freien Längsrandes (74) vor der Quermittelachse (30) des Inkontinenzartikels (2) in Richtung Bauchabschnitt (4) höchstens 0,30, insbesondere höchstens 0,25, insbesondere höchstens 0,20 beträgt und vorzugsweise mindestens 0,05, vorzugsweise mindestens 0,07, weiter vorzugsweise mindestens 0,10 beträgt.

6. Inkontinenzartikel nach einem der vorangehenden Ansprüche, **dadurch gekennzeichnet, dass** der jeweilige unbefestigte freie Längsrand (74) der beiden Cuffelemente (68) eine Gesamtlängserstreckung C3 aufweist, und mit einer Halbierung der Gesamtlängserstreckung C3 in ein vorderes Längsrand-Cuffsegment und in ein hinteres Längsrand-Cuffsegment aufteilbar ist, wobei ein Verhältnis einer innerhalb des vorderen Längsrand-Cuffsegments berechneten mittleren vorderen Cuffhöhe F(v) zu einer innerhalb des hinteren Längsrand-Cuffsegments berechneten mittleren hinteren Cuffhöhe F(h) mindestens 1,2, insbesondere mindestens 1,3, insbesondere mindestens 1,4 und insbesondere höchstens 2,0, weiter insbesondere höchstens 1,9, weiter insbesondere höchstens 1,8 beträgt.

7. Inkontinenzartikel nach einem der vorangehenden Ansprüche, **dadurch gekennzeichnet, dass** der Absorptionskörper entlang einer Längserstreckung beidseits durch Längskanten begrenzt ist, wobei die Längskanten des Absorptionskörpers (7) mit variierendem Abstand voneinander verlaufen, und der Abstand der Längskanten voneinander in Längsrichtung ein Maximum in einem vorderen Bereich des Absorptionskörpers aufweist.

8. Inkontinenzartikel nach einem der vorangehenden Ansprüche, **dadurch gekennzeichnet, dass** der Absorptionskörper eine Gesamtlänge S3 aufweist, und mit einer Halbierung der Gesamtlänge S3 in ein vorderes Absorptionskörpersegment und in ein hinteres Absorptionskörpersegment ein Verhältnis einer innerhalb des vorderen Absorptionskörpersegments berechneten mittleren vorderen Absorptionskörperbreite G(v) zu einer innerhalb des hinteren Absorptionskörpersegments berechneten mittleren hinteren Absorptionskörperbreite G(h) mindestens 1,2, insbesondere mindestens 1,3, insbesondere mindestens 1,4 und insbesondere höchstens 2,0, weiter insbesondere höchstens 1,9, weiter insbesondere höchstens 1,8 beträgt.

9. Inkontinenzartikel nach einem der vorstehenden Ansprüche, **dadurch gekennzeichnet, dass** ein Verhältnis einer Länge S2 des Absorptionskörpers (7) hinter der Quermittelachse (30) des Inkontinenzartikels (2) in Richtung Rückenabschnitt (6) zu einer Länge S1 des Absorptionskörpers (7) vor der Quermittelachse (30) des Inkontinenzartikels (2) in Richtung Bauchabschnitt (4) höchstens 0,25, insbesondere höchstens 0,20 beträgt und weiter vorzugsweise mindestens 0,05, vorzugsweise mindestens 0,10 beträgt.

10. Inkontinenzartikel nach einem der vorstehenden Ansprüche, **dadurch gekennzeichnet, dass** ein Verhältnis einer Länge S1 des Absorptionskörpers (7) vor der Quermittelachse (30) in Richtung Bauchabschnitt (4) zu einer Länge L1 des Inkontinenzartikels (2) ausgehend von der Quermittelachse (30) bis zu einer die Hüftöffnung (18) begrenzenden Hüftöffnungskante (18') des Bauchabschnitts (4) mindestens 0,6, insbesondere mindestens 0,7, weiter vorzugsweise höchstens 0,9, weiter vorzugsweise höchstens 0,8 beträgt.

11. Inkontinenzartikel nach einem der vorstehenden Ansprüche, **dadurch gekennzeichnet, dass** ein Verhältnis einer maximalen Höhe der Cuffelemente (68) ausgehend von der Cuffsockellinie (52) zum freien Längsrand (74) zu einer auf der Quermittelachse (30) des Inkontinenzartikels gemessenen Breite des Absorptionskörpers (7) mindestens 0,35, insbesondere mindestens 0,40, insbesondere mindestens 0,45 und insbesondere mindestens 0,50, weiter insbesondere höchstens 0,75, insbesondere höchstens 0,70, weiter insbesondere höchstens 0,65 beträgt.

12. Inkontinenzartikel nach einem der vorstehenden Ansprüche, **dadurch gekennzeichnet, dass** die Cuffsockellinien (52) zumindest abschnittsweise einer Kontur (45) des Absorptionskörpers (7) folgen und /oder die Cuffsockellinien (52) und die Längskanten des Absorptionskörpers (7) parallel verlaufen, weiter insbesondere, dass die Cuffsockellinien (52) zumindest abschnittsweise innerhalb der Kontur (45) des Absorptionskörpers (7) verlaufen.

13. Inkontinenzartikel nach einem der vorstehenden Ansprüche, **dadurch gekennzeichnet, dass** der Absorptionskörper (7) an einer vorderen Produkthälfte des Inkontinenzartikels einen Flächenanteil von mindestens 15%, insbesondere 18 - 35%, weiter insbesondere 20-35% aufweist und/oder dass der Absorptionskörper (7) an einer hinteren Produkthälfte des Inkontinenzartikels einen Flächenanteil von höchstens 10%, insbesondere höchstens 8%, insbesondere höchstens 6%, weiter insbesondere höchstens 4 %, und weiter insbesondere mindestens 0,5%, weiter insbesondere mindestens 1%, weitere insbesondere mindestens 1,5% aufweist.

14. Inkontinenzartikel nach einem der vorstehenden Ansprüche, **dadurch gekennzeichnet, dass** der Absorptionskörper (7) hinter der Quermittelachse (30) des Inkontinenzartikels in Richtung Rückenabschnitt in einer hinteren Produkthälfte eine Fläche aufweist, wobei ein Quotient von einer maximalen Breite des Absorptionskörpers in der hinteren Produkthälfte zu einer maximalen Länge des Absorptionskörpers in der hinteren Produkthälfte (entspricht S2) mindestens 1,0, weiter vorzugsweise mindestens 1,5, weitere vorzugsweise mindestens 1,7, weiter vorzugsweise mindestens 2,0, und weiter vorzugsweise höchstens 7,0, weiter vorzugsweise höchstens 6,0, weiter vorzugsweise höchstens 5,0 beträgt.

15. Anordnung von Inkontinenzartikel für Männer in Höschenform, wobei die Anordnung umfasst:
Einen ersten Inkontinenzartikel (2) mit einer Längsmittelachse (44) und einer ersten Quermittelachse (30) für die Aufnahme von Körperausscheidungen, wobei der erste Inkontinenzartikel einen in Längsrichtung voneinander beabstandeten vorderen Bauchabschnitt (4) und einen hinteren Rückenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, umfasst, und einen einen ersten Absorptionskörper (7) aufweisenden Schrittabschnitt (8), der sich in einer Längsrichtung (9) zwischen Bauchabschnitt (4) und Rückenabschnitt (6) erstreckt und an den Bauchabschnitt und den Rückenabschnitt in einem jeweiligen Überlappungsbereich unlösbar angefügt ist, und wobei der erste Absorptionskörper (7) so angeordnet ist, dass eine erste Quermittellinie (43) des ersten Absorptionskörpers zwischen der Quermittelachse (30) des ersten Inkontinenzartikels und einer die Hüftöffnung begrenzenden Hüftöffnungskante (18') des Bauchabschnitts (4) angeordnet ist, wobei der Schrittabschnitt (8), der Bauchabschnitt (4) und der Rückenabschnitt (6) gemeinsam Beinöffnungen (19) des Inkontinenzartikels begrenzen und mit eine seitliche Auslaufsperre bildenden und beidseits entlang der Längserstreckung des Absorptionskörpers verlaufenden Cuffelementen (68), die zumindest entlang einer Cuffsockellinie (52) an einer körperzugewandten Seite des Inkontinenzartikels festgelegt sind und einen ersten unbefestigten freien zumindest abschnittsweise in der Längsrichtung elastifizierten Längsrand (74) aufweisen, mit dem sie sich von der körperzugewandten Seite des Inkontinenzartikels emporerheben können und so die jeweilige seitliche Auslaufsperre bilden, wobei die Cuffelemente mit variierendem Abstand der Cuffsockellinien (52) voneinander geführt sind und einen zweiten Inkontinenzartikel (200) mit einer Längsmittelachse und einer zweiten Quermittelachse (243) für die Aufnahme von Körperausscheidungen, wobei der zweite Inkontinenzartikel einen in Längsrichtung voneinander beabstandeten vorderen Bauchabschnitt (4) und einen hinteren Rückenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, umfasst, und einen einen zweiten Absorptionskörper (207) aufweisenden Schrittabschnitt (8), der sich in einer Längsrichtung (9) zwischen Bauchabschnitt (4) und Rückenabschnitt (6) erstreckt und an den Bauchabschnitt und den Rückenabschnitt in einem jeweiligen Überlappungsbereich unlösbar angefügt ist, wobei der Schrittabschnitt (8),
der Bauchabschnitt (4) und der Rückenabschnitt (6) gemeinsam Beinöffnungen (19) des Inkontinenzartikels begrenzen und mit eine seitliche Auslaufsperre bildenden und beidseits entlang der Längserstreckung des Absorptionskörpers verlaufenden Cuffelementen, die zumindest entlang einer Cuffsockellinie (252) an einer körperzugewandten Seite des zweiten Inkontinenzartikels festgelegt sind und einen zweiten unbefestigten freien zumindest abschnittsweise in der Längsrichtung elastifizierten Längsrand aufweisen, mit dem sie sich von der körperzugewandten Seite des Inkontinenzartikels emporerheben können und so die jeweilige seitliche Auslaufsperre bilden,
wobei eine Fläche des ersten Absorptionskörpers (7) hinter der ersten Quermittelachse (30) des ersten Inkontinenzartikels (2) in Richtung auf den Rückenabschnitt des ersten Inkontinenzartikels (2) kleiner ist, als eine Fläche des zweiten Absorptionskörpers (207) hinter der zweiten Quermittelachse (230) des zweiten Inkontinenzartikels in Richtung auf den Rückenabschnitt des zweiten Inkontinenzartikels, **dadurch gekennzeichnet, dass** der erste Inkontinenzartikel (2) ein Inkontinenzartikel nach einem der Ansprüche 1 bis 14 ist.

16. Anordnung nach Anspruch 15, **dadurch gekennzeichnet, dass** die Fläche des ersten Absorptionskörpers (7) hinter der ersten Quermittelachse (30) beim ersten Inkontinenzartikel (2) um einen Faktor 2 - 9, vorzugsweise 3 - 9, weiter vorzugsweise 4 - 9, weiter vorzugsweise 4 - 8 kleiner ist als die Fläche des zweiten Absorptionskörpers (207) hinter der zweiten Quermittelachse (230) des zweiten Inkontinenzartikels (200) .

17. Anordnung nach einem der vorangehenden Ansprüche 15 - 16, **dadurch gekennzeichnet, dass** ein Verhältnis einer maximalen Höhe der Cuffelemente (68) ausgehend von der Cuffsockellinie (52) zum freien Längsrand (74) zu einer Breite des ersten Absorptionskörpers (7) gemessen auf der ersten Quermittelachse (30) beim ersten Inkontinenzartikel um den Faktor 1,05 - 1,50, insbesondere 1,10 -1,45, weiter insbesondere 1,15 - 1,40 größer ist als ein Verhältnis einer maximalen Höhe der Cuffelemente (68) ausgehend von der Cuffsockellinie (252) zum freien Längsrand (74) zu einer Breite des zweiten Absorptionskörpers (207) gemessen auf der zweiten Quermittelachse (230) beim zweiten Inkontinenzartikel (200).

18. Anordnung nach einem der vorangehenden Ansprüche 15 - 17, **dadurch gekennzeichnet, dass** ein Verhältnis einer Längserstreckung C4 des unbefestigten freien Längsrandes (74) hinter der Quermittelachse zu einer Längserstreckung C2 des freien Längsrandes vor der Quermittelachse, also C4/C2, des ersten Inkontinenzartikels (2) um einen Faktor von mindestens 3, weiter vorzugsweise mindestens 3,5, weiter vorzugsweise mindestens 4, weitere vorzugsweise höchstens 6, vorzugsweise höchstens 5,5 kleiner ist als ein Verhältnis C4 zu C2 des zweiten Inkontinenzartikels (200).

19. Anordnung nach einem der vorangehenden Ansprüche 15 - 18, **dadurch gekennzeichnet, dass** ein Verhältnis S2/S1 aus einer Erstreckung S2 des ersten Absorptionskörpers (7) hinter der ersten Quermittelachse (30) zu einer Erstreckung S1 des ersten Absorptionskörpers vor der ersten Quermittelachse (30) des ersten Inkontinenzartikels (2) zum Verhältnis S2/S1 einer Erstreckung S2 des zweiten Absorptionskörpers (207) hinter der zweiten Quermittelachse (230) zu einer Erstreckung S1 des zweiten Absorptionskörpers vor der zweiten Quermittelachse des zweiten Inkontinenzartikels (200) kleiner ist, insbesondere um den Faktor von mindestens 2, vorzugsweise mindestens 3, weiter vorzugweise höchstens 6, weiter vorzugsweise höchstens 5.

## Claims

1. Disposable incontinence article (2) in the form of underpants, the article comprising a longitudinal central axis (44) and a transverse central axis (30) for absorbing bodily excretions for preferred use by men, the article comprising a front abdominal portion (4) and a rear back portion (6) spaced apart from one another in a longitudinal direction (9), which portions are connected to one another by the manufacturer at side seam regions (14) on both sides to form a continuous abdominal and back band in the transverse or hip circumferential direction (16), which band comprises a hip opening (18) which is closed in the hip circumferential direction (16), and the article comprising a crotch portion (8) which has an absorption body (7) and extends in a longitudinal direction (9) between the abdominal portion (4) and the back portion (16) and is non-detachably attached to the abdominal portion (4) and the back portion (6) in a relevant overlapping region (36, 38), wherein the crotch portion (8), the abdominal portion (4) and the back portion (6) jointly delimit leg openings (19) of the incontinence article (2) and wherein the crotch portion (8) has cuff elements (68) on both sides which form a lateral leakage barrier and run on both sides along a longitudinal extension of the absorption body (7), which cuff elements are fixed at least along a cuff base line (52) on a side of the incontinence article (2) facing the body and have an unfixed, free longitudinal edge (74) which is elasticated at least partially in the longitudinal direction (9), with which edge the cuff elements (68) can be raised from the side of the incontinence article (2) facing the body and thus form the relevant lateral leakage barrier and wherein the absorption body (7) is arranged such that a transverse central line (43) of the absorption body (7) is arranged between the transverse central axis (30) of the incontinence article (2) and a hip opening edge (18') of the abdominal portion (4) which delimits the hip opening (18), and wherein the cuff elements (68) are guided at varying distances from one another between the cuff base lines (52), and wherein the absorption body (7) extends in the direction of the back portion (6) over the transverse central axis (30) of the incontinence article in the direction of a hip opening edge (18'') of the back portion (6), **characterized in that** a ratio of a length S2 of the absorption body (7) behind the transverse central axis (30) of the incontinence article (2), in the direction of the back portion (6), to a length S1 of the absorption body (7) in front of the transverse central axis (30) of the incontinence article (2), in the direction of the abdominal portion (4), is at most 0.30.

2. Incontinence article according to claim 1, **characterized in that** the relevant cuff base line (52) has a front cuff base line end portion (54) and/or a rear cuff base line end portion (56) running inwards in the transverse direction (16), in particular in an arc shape, in the direction of a longitudinal central axis (44) of the incontinence article (2) and thus in the direction of the free longitudinal edge (74) of the relevant cuff element (68), wherein this front and/or rear cuff base line end portion (54, 56) delimits, in the longitudinal direction (9), the region (80) of the cuff element (68) that can be raised.

3. Incontinence article according to any of the preceding claims, **characterized in that** a ratio of an average front cuff base line distance D(v) in a front cuff base line distance segment to an average rear cuff base line distance D(h) in a rear cuff base line distance segment is at least 1.2, in particular at least 1.3, in particular at least 1.4, and more particularly at most 2.3, more particularly at most 2.2, more particularly at most 2.1, more particularly at most 2.0, more particularly at most 1.9, more particularly at most 1.8, more particularly at most 1.7.

4. Incontinence article according to any of the preceding claims, **characterized in that** a ratio of a maximum distance of the cuff base lines (52) within a front half of the product in front of the transverse central axis of the incontinence article to a distance of the cuff base lines (52) measured on the transverse central axis (30) of the incontinence article is at least 1.3, in particular at least 1.4, and in particular at most 2.0, more particularly at most 1.8.

5. Incontinence article according to any of the preceding claims, **characterized in that** a ratio of a longitudinal extension C4 of the unfixed, free longitudinal edge (74) behind the transverse central axis (30) of the incontinence article (2), in the direction of the back portion (6), to a longitudinal extension C2 of the unfixed, free longitudinal edge (74) in front of the transverse central axis (30) of the incontinence article (2), in the direction of the abdominal portion (4), is at most 0.30, in particular at most 0.25, in particular at most 0.20 and preferably at least 0.05, preferably at least 0.07, more preferably at least 0.10.

6. Incontinence article according to any of the preceding claims, **characterized in that** each unfixed, free longitudinal edge (74) of the two cuff elements (68) has a total longitudinal extension C3, and can be divided into a front longitudinal edge cuff segment and a rear longitudinal edge cuff segment by halving the total longitudinal extension C3, wherein a ratio of an average front cuff height F(v) calculated within the front longitudinal edge cuff segment to an average rear cuff height F(h) calculated within the rear longitudinal edge cuff segment is at least 1.2, in particular at least 1.3, in particular at least 1.4 and in particular at most 2.0, more particularly at most 1.9, more particularly at most 1.8.

7. Incontinence article according to any of the preceding claims, **characterized in that** the absorption body is delimited along a longitudinal extension on both sides by longitudinal edges, wherein the longitudinal edges of the absorption body (7) run at varying distances from one another, and the distance of the longitudinal edges from one another in the longitudinal direction has a maximum in a front region of the absorption body.

8. Incontinence article according to any of the preceding claims, **characterized in that** the absorption body has a total length S3, and, with the total length S3 halved into a front absorption body segment and a rear absorption body segment, a ratio of an average front absorption body width G(v) calculated within the front absorption body segment to an average rear absorption body width G(h) calculated within the rear absorption body segment is at least 1.2, in particular at least 1.3, in particular at least 1.4 and in particular at most 2.0, more particularly at most 1.9, more particularly at most 1.8.

9. Incontinence article according to any of the preceding claims, **characterized in that** a ratio of a length S2 of the absorption body (7) behind the transverse central axis (30) of the incontinence article (2), in the direction of the back portion (6), to a length S1 of the absorption body (7) in front of the transverse central axis (30) of the incontinence article (2), in the direction of the abdominal portion (4), is at most 0.25, in particular at most 0.20, and more preferably at least 0.05, preferably at least 0.10.

10. Incontinence article according to any of the preceding claims, **characterized in that** a ratio of a length S1 of the absorption body (7) in front of the transverse central axis (30), in the direction of the abdominal portion (4), to a length L1 of the incontinence article (2) starting from the transverse central axis (30) to a hip opening edge (18') of the abdominal portion (4) that delimits the hip opening (18) is at least 0.6, in particular at least 0.7, more preferably at most 0.9, more preferably at most 0.8.

11. Incontinence article according to any of the preceding claims, **characterized in that** a ratio of a maximum height of the cuff elements (68), starting from the cuff base line (52) to the free longitudinal edge (74), to a width of the absorption body (7) measured on the transverse central axis (30) of the incontinence article is at least 0.35, in particular at least 0.40, in particular at least 0.45 and in particular at least 0.50, more particularly at most 0.75, in particular at most 0.70, more particularly at most 0.65.

12. Incontinence article according to any of the preceding claims, **characterized in that** the cuff base lines (52) at least partially follow a contour (45) of the absorption body (7), and/or the cuff base lines (52) and the longitudinal edges of the absorption body (7) run parallel, more particularly **in that** the cuff base lines (52) run at least partially within the contour (45) of the absorption body (7).

13. Incontinence article according to any of the preceding claims, **characterized in that** the absorption body (7), on a front half of the product of the incontinence article, has a surface area of at least 15%, in particular 18 - 35%, more particularly 20-35% and/or **in that** the absorption body (7), on a rear half of the product of the incontinence article, has a surface area of at most 10%, in particular at most 8%, in particular at most 6%, more particularly at most 4%, and more particularly at least 0.5%, more particularly at least 1%, more particularly at least 1.5%.

14. Incontinence article according to any of the preceding claims, **characterized in that** the absorption body (7) has a surface behind the transverse central axis (30) of the incontinence article in the direction of the back portion in a rear half of the product, wherein a quotient of a maximum width of the absorption body in the rear half of the product to a maximum length of the absorption body in the rear half of the product (corresponds to S2) is at least 1.0, more preferably at least 1.5, more preferably at least 1.7, more preferably at least 2.0, and more preferably at most 7.0, more preferably at most 6.0, more preferably at most 5.0.

15. Arrangement of incontinence articles for men in the form of underpants, wherein the arrangement comprises:
a first incontinence article (2) comprising a longitudinal central axis (44) and a first transverse central axis (30) for absorbing bodily excretions,
wherein the first incontinence article comprises a front abdominal portion (4) and a rear back portion (6) which are spaced apart from one another in the longitudinal direction, and which are connected to one another by the manufacturer at side seam regions (14) on both sides to form a continuous abdominal and back band in the transverse or hip circumferential direction (16), which band comprises a hip opening (18) which is closed in the hip circumferential direction, and a crotch portion (8) which has a first absorption body (7) and which extends in a longitudinal direction (9) between the abdominal portion (4) and the back portion (6) and is non-detachably attached to the abdominal portion and the back portion in a relevant overlapping region, and wherein the first absorption body (7) is arranged such that a first transverse central line (43) of the first absorption body is arranged between the transverse central axis (30) of the first incontinence article and a hip opening edge (18') of the abdominal portion (4) which delimits the hip opening, wherein the crotch portion (8), the abdominal portion (4) and the back portion (6) jointly delimit leg openings (19) of the incontinence article and have cuff elements (68) which form a lateral leakage barrier and run on both sides along the longitudinal extension of the absorption body, which cuff elements are fixed at least along a cuff base line (52) on a side of the incontinence article facing the body and have a first unfixed, free longitudinal edge (74) which is elasticated at least partially in the longitudinal direction, with which edge they can be raised from the side of the incontinence article facing the body and thus form the relevant lateral leakage barrier, wherein the cuff elements are guided at varying distances from one another between the cuff base lines (52),
and a second incontinence article (200) comprising a longitudinal central axis and a second transverse central axis (243) for receiving bodily excretions, wherein the second incontinence article comprises a front abdominal portion (4) and a rear back portion (6) which are spaced apart from one another in the longitudinal direction, and which are connected to one another by the manufacturer at side seam regions (14) on both sides to form a continuous abdominal and back band in the transverse or hip circumferential direction (16), which band comprises a hip opening (18) which is closed in the hip circumferential direction, and a crotch portion (8) which has a second absorption body (207) and which extends in a longitudinal direction (9) between the abdominal portion (4) and the back portion (6) and is non-detachably attached to the abdominal portion and the back portion in a relevant overlapping region, wherein the crotch portion (8), the abdominal portion (4) and the back portion (6) jointly delimit leg openings (19) of the incontinence article and have cuff elements which form a lateral leakage barrier and run on both sides along the longitudinal extension of the absorption body, which cuff elements are fixed at least along a cuff base line (252) on a side of the second incontinence article facing the body and have a second unfixed, free longitudinal edge which is elasticated at least partially in the longitudinal direction, with which edge they can be raised from the side of the incontinence article facing the body and thus form the relevant lateral leakage barrier,
wherein a surface of the first absorption body (7) behind the first transverse central axis (30) of the first incontinence article (2) in the direction of the back portion of the first incontinence article (2) is smaller than a surface of the second absorption body (207) behind the second transverse central axis (230) of the second incontinence article in the direction of the back portion of the second incontinence article, **characterized in that** the first incontinence article (2) is an incontinence article according to any of claims 1 to 14.

16. Arrangement according to claim 15, **characterized in that** the surface of the first absorption body (7) behind the first transverse central axis (30) of the first incontinence article (2) is smaller by a factor of 2 - 9, preferably 3 - 9, more preferably 4 - 9, more preferably 4 - 8 than the surface of the second absorption body (207) behind the second transverse central axis (230) of the second incontinence article (200) .

17. Arrangement according to any of the preceding claims 15 - 16, **characterized in that** a ratio of a maximum height of the cuff elements (68) starting from the cuff base line (52) to the free longitudinal edge (74) to a width of the first absorption body (7) measured on the first transverse central axis (30) in the first incontinence article is greater by a factor of 1.05 - 1.50, in particular 1.10 - 1.45, more particularly 1.15 - 1.40 than a ratio of a maximum height of the cuff elements (68) starting from the cuff base line (252) to the free longitudinal edge (74) to a width of the second absorption body (207) measured on the second transverse central axis (230) in the second incontinence article (200).

18. Arrangement according to any of the preceding claims 15 - 17, **characterized in that** a ratio of a longitudinal extension C4 of the unfixed, free longitudinal edge (74) behind the transverse central axis to a longitudinal extension C2 of the free longitudinal edge in front of the transverse central axis, i.e. C4/C2, of the first incontinence article (2) is smaller by a factor of at least 3, more preferably at least 3.5, more preferably at least 4, more preferably at most 6, preferably at most 5.5 than a ratio C4 to C2 of the second incontinence article (200).

19. Arrangement according to any of the preceding claims 15 - 18, **characterized in that** a ratio S2/S1 of an extension S2 of the first absorption body (7) behind the first transverse central axis (30) to an extension S1 of the first absorption body in front of the first transverse central axis (30) of the first incontinence article (2), with respect to the ratio S2/S1 of an extension S2 of the second absorption body (207) behind the second transverse central axis (230) to an extension S1 of the second absorption body in front of the second transverse central axis of the second incontinence article (200), is smaller, in particular by a factor of at least 2, preferably at least 3, more preferably at most 6, more preferably at most 5.

## Revendications

1. Article d'incontinence (2) jetable sous forme de culotte ayant un axe central longitudinal (44) et un axe central transversal (30) et destiné à recueillir les excrétions corporelles et à l'usage préféré par des hommes, comprenant une portion ventrale avant (4) et une portion dorsale arrière (6) qui sont espacées l'une de l'autre dans une direction longitudinale (9) et qui, pour la formation d'une bande ventrale et dorsale continue dans le sens transversal ou dans le sens du tour de hanches (16), avec une ouverture de hanche (18) fermée dans le sens du tour de hanches (16), sont reliées entre elles par le fabricant au niveau de zones de couture latérale (14) situées de part et d'autre, et comprenant une portion d'entrejambe (8) présentant un corps absorbant (7), qui s'étend dans une direction longitudinale (9) entre la portion ventrale (4) et la portion dorsale (6) et qui est rapportée de manière inamovible sur la portion ventrale (4) et sur la portion dorsale (6) dans une zone de chevauchement (36, 38) respective, dans lequel ladite portion d'entrejambe (8), ladite portion ventrale (4) et ladite portion dorsale (6) délimitent ensemble des ouvertures de jambe (19) de l'article d'incontinence (2), et dans lequel la portion d'entrejambe (8) comprend des éléments de manchette (68) qui forment une barrière anti-fuites latérale des deux côtés et s'étendent des deux côtés le long d'une extension longitudinale du corps absorbant (7) et qui sont fixés au moins le long d'une ligne de base de manchette (52) sur un côté de l'article d'incontinence (2), qui est tourné vers le corps, et présentent un bord longitudinal libre (74) non fixé qui est élastifié, au moins par sections, dans la direction longitudinale (9) et avec lequel les éléments de manchette (68) peuvent s'étendre vers le haut depuis le côté de l'article d'incontinence (2), qui est tourné vers le corps, et forment ainsi la barrière anti-fuites latérale respective, et dans lequel le corps absorbant (7) est disposé de telle sorte qu'un une ligne centrale transversale (43) du corps absorbant (7) est disposée entre l'axe central transversal (30) de l'article d'incontinence (2) et un bord d'ouverture de hanche (18') de la portion ventrale (4), qui délimite l'ouverture de hanche (18), et dans lequel les éléments de manchette (68) sont guidés avec une distance variable entre les lignes de base de manchette (52) les unes par rapport aux autres, et dans lequel le corps absorbant (7) s'étend en direction de la portion dorsale (6) sur l'axe central transversal (30) de l'article d'incontinence en direction d'un bord d'ouverture de hanche (18'') de la portion dorsale (6), **caractérisé par le fait qu'**un rapport d'une longueur S2 du corps absorbant (7) derrière l'axe central transversal (30) de l'article d'incontinence (2) en direction de la portion dorsale (6) à une longueur S1 du corps absorbant (7) devant l'axe central transversal (30) de l'article d'incontinence (2) en direction de la portion ventrale (4) est de 0,3 tout au plus.

2. Article d'incontinence selon la revendication 1, **caractérisé par le fait que** la ligne de base de manchette (52) respective présente une section d'extrémité avant de ligne de base de manchette (54) et/ou section d'extrémité arrière de ligne de base de manchette (56), qui s'étend dans le sens transversal (16), en particulier en forme d'arc vers l'intérieur en direction d'un axe central longitudinal (44) de l'article d'incontinence (2) et ainsi en direction du bord longitudinal libre (74) de l'élément de manchette (68) respectif, dans lequel cette section d'extrémité avant et/ou arrière de ligne de base de manchette (54, 56) délimite dans la direction longitudinale (9) la zone (80) de l'élément de manchette (68), qui peut s'étendre vers le haut.

3. Article d'incontinence selon l'une quelconque des revendications précédentes, **caractérisé par le fait qu'**un rapport d'une distance moyenne avant de ligne de base de manchette D (v) dans un segment avant de distance de ligne de base de manchette à une distance moyenne arrière de ligne de base de manchette D (h) dans un segment arrière de distance de ligne de base de manchette est d'au moins 1,2, en particulier d'au moins 1,3, en particulier d'au moins 1,4, et encore en particulier de 2,3 tout au plus, encore en particulier de 2,2 tout au plus, encore en particulier de 2,1 tout au plus, encore en particulier de 2,0 tout au plus, encore en particulier de 1,9 tout au plus, encore en particulier de 1,8 tout au plus, encore en particulier de 1,7 tout au plus.

4. Article d'incontinence selon l'une quelconque des revendications précédentes, **caractérisé par le fait qu'**un rapport d'une distance maximale des lignes de base de manchette (52) à l'intérieur d'une moitié avant de produit devant l'axe central transversal de l'article d'incontinence à une distance des lignes de base de manchette (52) mesurées sur l'axe central transversal (30) de l'article d'incontinence est d'au moins 1,3, en particulier d'au moins 1,4, et en particulier de 2,0 tout au plus, encore en particulier de 1,8 tout au plus.

5. Article d'incontinence selon l'une quelconque des revendications précédentes, **caractérisé par le fait qu'**un rapport d'une extension longitudinale C4 du bord longitudinal libre non fixé (74) derrière l'axe central transversal (30) de l'article d'incontinence (2) en direction de la portion dorsale (6) à une extension longitudinale C2 du bord longitudinal libre non fixé (74) devant l'axe central transversal (30) de l'article d'incontinence (2) en direction de la portion ventrale (4) est de 0,30 tout au plus, en particulier de 0,25 tout au plus, en particulier de 0,20 tout au plus et de préférence d'au moins 0,05, de préférence d'au moins 0,07, encore de préférence d'au moins 0,10.

6. Article d'incontinence selon l'une quelconque des revendications précédentes, **caractérisé par le fait que** le bord longitudinal libre non fixé (74) respectif des deux éléments de manchette (68) présente une extension longitudinale totale C3 et peut être divisé, avec une division par deux de l'extension longitudinale totale C3, en un segment avant de manchette de bord longitudinal et un segment arrière de manchette de bord longitudinal, dans lequel un rapport d'une hauteur de manchette avant moyenne F (v) calculée à l'intérieur du segment avant de manchette de bord longitudinal à une hauteur de manchette arrière moyenne F (h) calculée à l'intérieur du segment arrière de manchette de bord longitudinal est d'au moins 1,2, en particulier d'au moins 1,3, en particulier d'au moins 1,4 et en particulier de 2,0 tout au plus, encore en particulier de 1,9 tout au plus, encore en particulier de 1,8 tout au plus.

7. Article d'incontinence selon l'une quelconque des revendications précédentes, **caractérisé par le fait que** le corps absorbant est délimité de part et d'autre, le long d'une extension longitudinale, par des bords longitudinaux, dans lequel les bords longitudinaux du corps absorbant (7) s'étendent à une distance variable les uns des autres, et la distance entre les bords longitudinaux les uns des autres dans la direction longitudinale présente un maximum dans une zone avant du corps absorbant.

8. Article d'incontinence selon l'une quelconque des revendications précédentes, **caractérisé par le fait que** le corps absorbant présente une longueur totale S3, et que, avec une division par deux de la longueur totale S3 en un segment avant de corps absorbant et en un segment arrière de corps absorbant, un rapport d'une largeur moyenne avant de corps absorbant G (v) calculée à l'intérieur du segment avant de corps absorbant à une largeur moyenne arrière de corps absorbant G (h) calculée à l'intérieur du segment arrière de corps absorbant est d'au moins 1,2, en particulier d'au moins 1,3, en particulier d'au moins 1,4 et en particulier de 2,0 tout au plus, encore en particulier de 1,9 tout au plus, encore en particulier de 1,8 tout au plus.

9. Article d'incontinence selon l'une quelconque des revendications précédentes, **caractérisé par le fait qu'**un rapport d'une longueur S2 du corps absorbant (7) derrière l'axe central transversal (30) de l'article d'incontinence (2) en direction de la portion dorsale (6) à une longueur S1 du corps absorbant (7) devant l'axe central transversal (30) de l'article d'incontinence (2) en direction de la portion ventrale (4) est de 0,25 tout au plus, en particulier de 0,20 tout au plus et encore de préférence d'au moins 0,05, de préférence d'au moins 0,10.

10. Article d'incontinence selon l'une quelconque des revendications précédentes, **caractérisé par le fait qu'**un rapport d'une longueur S1 du corps absorbant (7) devant l'axe central transversal (30) en direction de la portion ventrale (4) à une longueur L1 de l'article d'incontinence (2) à partir de l'axe central transversal (30) jusqu'à un bord d'ouverture de hanche (18') de la portion ventrale (4), qui délimite l'ouverture de hanche (18) est d'au moins 0,6, en particulier d'au moins 0,7, encore de préférence de 0,9 tout au plus, encore de préférence de 0,8 tout au plus.

11. Article d'incontinence selon l'une quelconque des revendications précédentes, **caractérisé par le fait qu'**un rapport d'une hauteur maximale des éléments de manchette (68) à partir de la ligne de base de manchette (52) jusqu'au bord longitudinal libre (74) à une largeur du corps absorbant (7) mesurée sur l'axe central transversal (30) de l'article d'incontinence est d'au moins 0,35, en particulier d'au moins 0,40, en particulier d'au moins 0,45 et en particulier d'au moins 0,50, encore en particulier de 0,75 tout au plus, en particulier de 0,70 tout au plus, encore en particulier de 0,65 tout au plus.

12. Article d'incontinence selon l'une quelconque des revendications précédentes, **caractérisé par le fait que** les lignes de base de manchette (52) suivent au moins par sections un contour (45) du corps absorbant (7) et/ou que les lignes de base de manchette (52) et les bords longitudinaux du corps absorbant (7) s'étendent parallèlement, encore en particulier que les lignes de base de manchette (52) s'étendent au moins par sections à l'intérieur du contour (45) du corps absorbant (7).

13. Article d'incontinence selon l'une quelconque des revendications précédentes, **caractérisé par le fait que**, sur une moitié avant de produit de l'article d'incontinence, le corps absorbant (7) présente une proportion de surface d'au moins 15 %, en particulier de 18 à 35 %, encore en particulier de 20 à 35 % et/ou que, sur une moitié arrière de produit de l'article d'incontinence, le corps absorbant (7) présente une proportion de surface de 10 % tout au plus, en particulier de 8 % tout au plus, en particulier de 6 % tout au plus, encore en particulier de 4 % tout au plus, et encore en particulier d'au moins 0,5 %, encore en particulier d'au moins 1 %, encore en particulier d'au moins 1,5 %.

14. Article d'incontinence selon l'une quelconque des revendications précédentes, **caractérisé par le fait que** le corps absorbant (7) présente une surface derrière l'axe central transversal (30) de l'article d'incontinence, en direction de la portion dorsale, dans une moitié arrière de produit, dans lequel un quotient d'une largeur maximale du corps absorbant dans la moitié arrière de produit à une longueur maximale du corps absorbant dans la moitié arrière de produit (correspond à S2) est d'au moins 1,0, encore de préférence d'au moins 1,5, encore de préférence d'au moins 1,7, encore de préférence d'au moins 2,0, et encore de préférence de 7,0 tout au plus, encore de préférence de 6,0 tout au plus, encore de préférence de 5,0 tout au plus.

15. Ensemble d'article d'incontinence pour hommes sous forme de culotte, ledit ensemble comprenant:
un premier article d'incontinence (2) ayant un axe central longitudinal (44) et un premier axe central transversal (30) et destiné à recueillir les excrétions corporelles, dans lequel ledit premier article d'incontinence (2) comprend une portion ventrale avant (4) et une portion dorsale arrière (6) qui sont espacées l'une de l'autre dans une direction longitudinale (9) et qui, pour la formation d'une bande ventrale et dorsale continue dans le sens transversal ou dans le sens du tour de hanches (16), avec une ouverture de hanche (18) fermée dans le sens du tour de hanches, sont reliées entre elles par le fabricant au niveau de zones de couture latérale (14) situées de part et d'autre, et comprenant une portion d'entrejambe (8) présentant un premier corps absorbant (7), qui s'étend dans une direction longitudinale (9) entre la portion ventrale (4) et la portion dorsale (6) et qui est rapportée de manière inamovible sur la portion ventrale (4) et sur la portion dorsale dans une zone de chevauchement respective, et dans lequel le premier corps absorbant (7) est disposé de telle sorte qu'une première ligne centrale transversale (43) du premier corps absorbant est disposée entre l'axe central transversal (30) du premier article d'incontinence et un bord d'ouverture de hanche (18') de la portion ventrale (4), qui délimite l'ouverture de hanche, dans lequel ladite portion d'entrejambe (8), ladite portion ventrale (4) et ladite portion dorsale (6) délimitent ensemble des ouvertures de jambe (19) de l'article d'incontinence, et avec des éléments de manchette (68) qui forment une barrière anti-fuites latérale et s'étendent des deux côtés le long de l'extension longitudinale du corps absorbant et qui sont fixés au moins le long d'une ligne de base de manchette (52) sur un côté de l'article d'incontinence, qui est tourné vers le corps, et présentent un premier bord longitudinal libre (74) non fixé qui est élastifié, au moins par sections, dans la direction longitudinale et avec lequel ils peuvent s'étendre vers le haut depuis le côté de l'article d'incontinence, qui est tourné vers le corps, et forment ainsi la barrière anti-fuites latérale respective, dans lequel les éléments de manchette (68) sont guidés avec une distance variable entre les lignes de base de manchette (52) les unes par rapport aux autres, et
un deuxième article d'incontinence (200) ayant un axe central longitudinal et un deuxième axe central transversal (243) et destiné à recueillir les excrétions corporelles, dans lequel ledit deuxième article d'incontinence comprend une portion ventrale avant (4) et une portion dorsale arrière (6) qui sont espacées l'une de l'autre dans la direction longitudinale (9) et qui, pour la formation d'une bande ventrale et dorsale continue dans le sens transversal ou dans le sens du tour de hanches (16), avec une ouverture de hanche (18) fermée dans le sens du tour de hanches, sont reliées entre elles par le fabricant au niveau de zones de couture latérale (14) situées de part et d'autre, et comprenant une portion d'entrejambe (8) présentant un deuxième corps absorbant (207), qui s'étend dans une direction longitudinale (9) entre la portion ventrale (4) et la portion dorsale (6) et qui est rapportée de manière inamovible sur la portion ventrale et sur la portion dorsale dans une zone de chevauchement respective, dans lequel ladite portion d'entrejambe (8), ladite portion ventrale (4) et ladite portion dorsale (6) délimitent ensemble des ouvertures de jambe (19) de l'article d'incontinence, et avec des éléments de manchette qui forment une barrière anti-fuites latérale et s'étendent des deux côtés le long de l'extension longitudinale du corps absorbant et qui sont fixés au moins le long d'une ligne de base de manchette (252) sur un côté du deuxième l'article d'incontinence, qui est tourné vers le corps, et présentent un deuxième bord longitudinal libre non fixé qui est élastifié, au moins par sections, dans la direction longitudinale et avec lequel ils peuvent s'étendre vers le haut depuis le côté de l'article d'incontinence, qui est tourné vers le corps, et forment ainsi la barrière anti-fuites latérale respective,
dans lequel une surface du premier corps absorbant (7) derrière le premier axe central transversal (30) du premier article d'incontinence (2) en direction de la portion dorsale du premier article d'incontinence (2) est plus petite qu'une surface du deuxième corps absorbant (207) derrière le deuxième axe central transversal (230) du deuxième article d'incontinence en direction de la portion dorsale du deuxième article d'incontinence, **caractérisé par le fait que** le premier article d'incontinence (2) est un article d'incontinence selon l'une quelconque des revendications 1 à 14.

16. Ensemble selon la revendication 15, **caractérisé par le fait que** la surface du premier corps absorbant (7) derrière le premier axe central transversal (30) dans le premier article d'incontinence (2) est plus petite d'un facteur de 2 à 9, de préférence de 3 à 9, encore de préférence de 4 à 9, encore de préférence de 4 à 8, que la surface du deuxième corps absorbant (207) derrière le deuxième axe central transversal (230) du deuxième article d'incontinence (200).

17. Ensemble selon l'une quelconque des revendications précédentes 15 à 16, **caractérisé par le fait qu'**un rapport d'une hauteur maximale des éléments de manchette (68) à partir de la ligne de base de manchette (52) jusqu'au bord longitudinal libre (74) à une largeur du premier corps absorbant (7) mesurée sur le premier axe central transversal (30) dans le premier article d'incontinence est plus grand du facteur de 1,05 à 1,50, en particulier de 1,10 à 1,45, en particulier encore de 1,15 à 1,40, qu'un rapport d'une hauteur maximale des éléments de manchette (68) à partir de la ligne de base de manchette (252) jusqu'au bord longitudinal libre (74) à une largeur du deuxième corps absorbant (207) mesurée sur le deuxième axe central transversal (230) dans le deuxième article sur l'incontinence (200).

18. Ensemble selon l'une quelconque des revendications précédentes 15 à 17, **caractérisé par le fait qu'**un rapport d'une extension longitudinale C4 du bord longitudinal libre non fixé (74) derrière l'axe central transversal à une extension longitudinale C2 du bord longitudinal libre devant l'axe central transversal, donc C4/C2, du premier article d'incontinence (2) est plus petit d'un facteur d'au moins 3, encore de préférence d'au moins 3,5, encore de préférence d'au moins 4, encore de préférence de 6 tout au plus, de préférence de 5,5 tout au plus, qu'un rapport de C4 à C2 du deuxième article d'incontinence (200).

19. Ensemble selon l'une quelconque des revendications précédentes 15 à 18, **caractérisé par le fait qu'**un rapport S2/S1 d'une extension S2 du premier corps absorbant (7) derrière le premier axe central transversal (30) à une extension S1 du premier corps absorbant devant le premier axe central transversal (30) du premier article d'incontinence (2) au rapport S2/S1 d'une extension S2 du deuxième corps absorbant (207) derrière le deuxième axe central transversal (230) à une extension S1 du deuxième corps absorbant devant le deuxième axe central transversal du deuxième article d'incontinence (200) est plus petit, en particulier du facteur d'au moins 2, de préférence d'au moins 3, encore de préférence de 6 tout au plus, encore de préférence de 5 tout au plus.
